# NEUE EUROPÄISCHE PATENTSCHRIFT

(11) **EP 0 399 059 B2**
(45) Veröffentlichungstag und Bekanntmachung des Hinweises auf die Entscheidung über den Einspruch: **20.08.2003**
(45) Hinweis auf die Patenterteilung: 27.10.1993
(21) Anmeldenummer: 89109157.1
(22) Anmeldetag: 22.05.1989
(51) Int. Cl.: A61N 1/36, A61N 1/08

(54) **Implantierbares medizinisches Gerät zur Detektion von bezüglich einer physiologischen Funktion auftretenden Ereignissen mit einstellbarer Empfindlichkeit und Verfahren zum Betrieb eines solchen Gerätes**
Implantable medical device with adjustable sensitivity to detect an event relating to a physiological function, and process for using same
Appareil médical implantable pour détecter un évènement relatif à une fonction physiologique à sensibilité réglable et son procédé d'utilisation

(43) Veröffentlichungstag der Anmeldung: 28.11.1990
(73) Patentinhaber: St. Jude Medical AB, 175 84 Järfälla (SE)
(72) Erfinder: Högnelid, Kurt, Dipl.-Ing., S-172 35 Sundbyberg (SE)
(74) Vertreter: Harrison, Michael Charles

(56) Entgegenhaltungen:
- EP-A- 0 220 916
- DE-A- 3 629 587
- DE-B- 2 717 530
- US-A- 4 552 154
- US-A- 4 766 902
- US-A- 4 768 511
- US-A- 4 773 401

## Beschreibung

Die Erfindung betrifft ein in den Körper eines Lebewesens implantierbares medizinisches Gerät mit einer Detektoreinrichtung zur Detektion von bezüglich einer physiologischen Funktion auftretenden Ereignissen, wobei die Ereignisse natürliche Herzschläge sind und wobei die Empfindlichkeit der Detektoreinrichtung einstellbar ist. Die Erfindung betrifft ausserdem ein Verfahren zum Betrieb eines derartigen Gerätes. Dabei soll der Begriff Empfindlichkeit derart verstanden werden, dass es sich bei der Empfindlichkeit um einen Schwellwert handelt, den der oder die bei der Detektion berücksichtigten Signalparameter eines der physiologischen Funktion entsprechenden Signales, z.B. die Amplitude und/oder die Steilheit eines elektrischen Signals, überschreiten müssen, um zu einer Detektion zu führen. Dabei entspricht einer hohen Empfindlichkeit ein niedriger Schwellwert, während einer niedrigen Empfindlichkeit ein hoher Schwellwert entspricht.

Bei Geräten der eingangs genannten Art tritt das Problem auf, dass man einerseits die Empfindlichkeit möglichst hoch einstellen möchte, um zu gewährleisten, dass tatsächlich auch alle auftretenden Erignisse detektiert werden, und man andererseits eine bestimmte Empfindlichkeit nicht überschreiten kann, ohne dass die Gefahr besteht, dass die Detektoreinrichtung dem der physiologischen Funktion entsprechenden Signal überlagerte Störungen fälschlicherweise als Ereignisse delektiert. In der Praxis wird die Empfindlichkeit daher in der Regel auf einen Wert eingestellt, der einen Kompromiss bezüglich der genannten Kriterien darstellt.

Aus der Druckschrift "Pulse Generator 704 - Physician's Manual", Siemens-Elema AB, Solna, Schweden, März 1985, ist ein Gerät der eingangs genannten Art bekannt, das als Herzschrittmacher ausgebildet ist. Dieser Herzschrittmacher gestattet es, mittels eines geeigneten Aufzeichnungsgerätes ein sogenanntes intrakardielles Elektrokardiogramm (IEKG) aufzuzeichnen, in dem dasjenige Signal dargestellt ist, das der Detektoreinrichtung des Herzschrittmachers zugeführt ist. Bei der Aufzeichnung dieses Signals werden, falls eine entsprechende Funktion des Herzschrittmachers aktiviert ist, diejenigen Ereignisse markiert, die die Detektoreinrichtung des Herzschrittmachers als natürliche Herzschläge detektiert. Der behandelnde Arzt ist somit in der Lage, die Detektoreinrichtung zunächst auf eine Empfindlichkeit einzustellen, in der alle Ereignisse in dem aufgezeichneten Signal, die der Arzt als natürliche Herzschläge erkennt, zu einer Detektion führen. Anschliessend stellt der behandelnde Arzt die Empfindlichkeit der Detektoreinrichtung auf einen um ein Sicherheits-Marginal höheren Wert ein, den er in Abhängigkeit von den individuellen Gegebenheiten des jeweiligen Patienten wählt. Da sich der jeweilige Patient nach Abschluss des mit der Implantation verbundenen Klinikaufenthalts nur in grösseren Abständen bei dem behandelnden Arzt vorstellt, besteht die Gefahr, dass sich, z.B. infolge von Veränderungen der Lage der die der elektrischen Aktivität des Herzens entsprechenden Signale zu der Detektoreinrichtung leitenden endokardiellen Elektrode, Verhältnisse ergeben, denen die vom behandelnden Arzt gewählte Einstellung der Empfindlichkeit der Detektoreinrichtung nicht mehr entspricht. Dies kann zu Fehlfunktionen führen, unabhängig davon, ob die vorhandene Einstellung der Empfindlichkeit für die veränderten Verhältnisse zu hoch oder niedrig ist. Im ersten Falle besteht die Gefahr, dass Störsignale fälschlicherweise als natürliche Herzschläge detektiert werden und demzufolge eine an sich erforderliche Stimulation des Herzens unterbleibt. Im zweiten Falle besteht die Gefahr, dass nicht alle auftretenden natürlichen Herzschläge detektiert werden und demzufolge das Herz des Patienten unnötigerweise stimuliert wird. Beides ist gleichermassen unerwünscht, da zumindest das Wohlbefinden des Patienten beeinträchtigt wird.

Gemäß US-A-4 766 902 und US-A-4 768 511 erfolgt die Empfindlichkeitsregelung zweier Detektoreinrichtung in der Weise, daß im normalen Betrieb nur eine Detektoreinrichtung die ausgewählte Herzaktivität erfasst.

Des weiteren offenbart die EP-A-0 222 681 einen herzschrittmacher, bei dem mindestens zwei Meßwertaufnehmer für von der körperlichen Belastung abhängige Signale vorgesehen sind, sowie Schaltmittel zur Zusammenführung dieser Signale. Die betreffenden physiologischen Signale können durch zusätzliche Meßwertaufnehmer erfasst und als Kontrollsignale benutzt werden.

Der Erfindung liegt die Aufgabe zugrunde, ein Gerät der eingangs genannten Art so auszubilden, dass die Detektoreinrichtung ohne Eingriffe des behandelnden Arztes stets mit der erforderlichen Empfindlichkeit arbeitet. Der Erfindung liegt ausserdem die Aufgabe zugrunde, ein Verfahren zum Betrieb eines Gerätes der eingangs genannten Art anzugeben, das auf für den Patienten sichere und energiesparende Weise eine Anpassung der Empfindlichkeit der Detektoreinrichtung an sich verändernde Verhältnisse ermöglicht.

Der das Gerät betreffende Teil der Aufgabe wird durch ein Gerät mit den Merkmalen des Patentanspruches 1 gelöst. Demnach wird also die Empfindlichkeit der ersten Detektoreinrichtung, die die eigentliche Detektoreinrichtung darstellt, mittels derer die natürlichen Herzschläge detektiert werden, anhand einer nur als Referenz vorgesehenen zweiten Detektoreinrichtung eingestellt. Dabei ist es zweckmässig, wenn die zweite Detektoreinrichtung eine höhere Empfindlichkeit als die jeweils eingestellte Empfindlichkeit der ersten Detektoreinrichtung aufweist. Da unter diesen Voraussetzungen die Empfindlichkeiten der beiden Detektoreinrichtungen unter Umständen nicht mehr direkt ihrem Zahlenwert nach verglichen werden können, soll hier unter gleicher Empfindlichkeit verstanden werden, dass ein Ereignis, das mit der ersten Detektoreinrichtung gerade noch detektiert werden kann, auch mit der zweiten Detektoreinrichtung detektiert werden kann. Eine grössere Empfindlichkeit der zweiten Detektoreinrichtung bedeutet dann, dass diese noch Ereignisse detektiert, die die erste Detektoreinrichtung nicht mehr zu detektieren vermag.

Die Erfindung gemäß Patentanspruch 1 besitzt den Vorteil, dass die Detektion eines Ereignisses mittels der zweiten Detektoreinrichtung einige Millisekunden nach der Detektion des gleichen Ereignisses mittels der ersten Detektoreinrichtung erfolgt. Da somit die Detektionen nicht gleichzeitig auftreten, ist es leicht feststellbar, ob ein mittels der zweiten Detektoreinrichtung detektiertes Ereignis auch mittels der ersten Detektoreinrichtung detektiert wird, da jeweils nur geprüft werden muss, ob kurzzeitig vor der Detektion eines Ereignisses mittels der zweiten Detektoreinrichtung eine Detektion auch mittels der ersten Detektoreinrichtung erfolgt ist. Die zur Herstellung einer elektrischen Verbindung der zweiten Detektoreinrichtung mit dem Herzen des Lebewesens erforderliche Elektrode kann übrigens gleichzeitig dazu dienen, der ersten Detektoreinrichtung dass der elektrischen Aktivität des Herzens entsprechende Signal zuzuführen.

Der Patentanspruch 2 hat eine bevorzugte Ausführungsform der Erfindung zum Gegenstand. Bei dieser Ausführungsform liegt die eingestellte Empfindlichkeit jeweils um ein in Abhängigkeit von den bei dem jeweiligen Patienten vorliegenden individuellen Gegebenheiten wählbares Sicherheits-Marginal höher, als der zur Detektion aller mittels der zweiten Detektoreinrichtung detektierte Ereignisse erforderliche Mindestwert der Empfindlichkeit. Somit ist unter allem Umständen ein sicherer Betrieb des erfindungsgemässen Gerätes gewährleistet.»

Die im Patentanspruch 5 angegebene Variante der Erfindung bietet den Vorteil, dass das der Impedanz des Herzens entsprechende Signal durch eine reine Wechselstrom-Messung gewonnen wird. Störende Einflüsse, wie sie etwa dadurch auftreten können, dass das Gewebe des Herzmuskels kurz nach einem Stimulationsimpuls ein von seinem Ruhepotential abweichendes Potential führt, sind dadurch vermieden. Ausserdem sind, falls die Messung mit einem Wechselstrom erfolgt, der eine sehr hohe Frequenz, z.B. mehrere Kilohertz, besitzt, Störungen der ersten Detektoreinrichtung ausgeschlossen, da diese auf derart hohe Frequenzen nicht anspricht.

Der das Verfahren betreffende Teil der Aufgabe ist erfindungsgemäss durch das im Patentanspruch 7 angegebene Verfahren gelöst. Demnach erfolgt also die Einstellung der Empfindlichkeit nicht kontinuierlich, sondern jeweils nur zu aufeinanderfolgenden ersten Zeitpunkten, wobei das Zeitintervall zwischen zwei aufeinanderfolgenden ersten Zeitpunkten beispielsweise eine Dauer in der Grössenordnung von Stunden besitzen kann. Die zweite Detektoreinrichtung braucht also nur in grösseren Zeitabständen in Betrieb genommen zu werden, was sich günstig auf den Energiebedarf des Geräts und damit die Lebensdauer einer das Gerät speisenden Batterie auswirkt. Da sprunghafte Änderungen der Empfindlichkeit der ersten Detektoreinrichtung in der Regel nicht erforderlich sind, sind durch die beschriebene intermittierende Betriebsweise keine nachteiligen Folgen für den Patienten zu befürchten. Dennoch kann im Falle von etwaigen Störungen rasch eine Korrektur der Empfindlichkeit der ersten Detektorschaltung erfolgen, da gemäss Patentanspruch 8 zu zweiten Zeitpunkten, zwischen denen beispielsweise ein Zeitintervall mit einer Dauer in der Grössenordnung von Minuten liegt, überprüft wird, ob die eingestellte Empfindlichkeit der ersten Detektoreinrichtung hoch genug ist, um mittels der zweiten Detektoreinrichtung detektierte Ereignisse detektieren zu können.

Die Verfahren gemäss den Patentansprüchen 9 bis 12 bieten den Vorteil, dass eine Empfindlichkeit der ersten Detektoreinrichtung eingestellt wird, die sich am Mindestwert der Empfindlichkeit der ersten Detektoreinrichtung orientiert, bei dem diese jedes mittels der zweiten Detektoreinrichtung detektierte Ereignis detektiert. Hierdurch ist gewährleistet, dass die Empfindlichkeit der ersten Detektoreinrichtung nicht höher ist, als dies, gegebenenfalls unter Berücksichtigung von Sicherheits-Marginalen, unbedingt erforderlich ist. Es ist also sichergestellt, dass einerseits Fehldetektionen vermieden sind und andererseits alle Ereignisse mittels der ersten Detektoreinrichtung detektiert werden.

Die Verfahren gemäss den Patentansprüchen 13 bis 16 betreffen die Ermittlung des Mindestwertes der Empfindlichkeit der ersten Detektoreinrichtung. Dabei ist für den Fall einer Erhöhung des Mindestwertes der Empfindlichkeit sichergestellt, dass der höhere Mindestwert der Empfindlichkeit rasch gefunden werden kann, so dass die Zahl von Ereignissen, die mittels der ersten Detektoreinrichtung nicht detektiert werden können, auf ein Mindestmass beschränkt ist. Für den Fall einer Verringerung des Mindestwertes der Empfindlichkeit wird der geringere Mindeswert bei grösseren Änderungen schrittweise zu mehreren ersten Zeitpunkten ermittelt. Dies bietet den Vorteil, dass bei nur kurzzeitigen Änderungen des Mindestwertes der Empfindlichkeit die Empfindlichkeit der ersten Detektoreinrichtung nicht so weit gesenkt wird, dass die Gefahr besteht, dass Ereignisse nicht detektiert werden können.

Mit dem Verfahren gemäss Patentanspruch 17 wird der Vorteil erziehlt, dass während der selbsttätigen Einstellung der Empfindlichkeit der ersten Detektoreinrichtung von zwei aufeinanderfolgenden Ereignissen wenigstens eines detektiert wird, so dass auch während der selbsttätigen Einstellung der Empfindlichkeit der ersten Detektoreinrichtung eine ausreichende Stimulation der physiologischen Funktion sichergestellt ist.

Die Erfindung wird nachfolgend anhand der beigefügten Zeichnungen näher erläutert. Es zeiqen:
- Fig. 1: ein Blockschaltbild eines als Herzschrittmacher ausgebildeten erfindungsgemässen Gerätes,
- Fig. 2: ein Blockschaltbild der zweiten Detektoreinrichtung des Herzschrittmachers gemäss Fig. 1,
- Fig. 3: für eine Anzahl natürliche Herzschläge das der elektrischen Aktivität des Herzens entsprechende Signal sowie das entsprechende der Impedanz des Herzens entsprechende Signal,
- Fig. 4: ein ein Betriebsverfahren zur selbsttätigen Einstellung des Energiegehaltes der Stimulationsimpulse des Herzschrittmachers gemäss Fig. 1 verdeutlichendes Flussdiagram, und
- Fig. 5: ein ein Betriebsverfahren zur selbsttätigen Einstellung der Empfindlichkeit der ersten Detektoreinrichtung des Herzschrittmachers gemäss Fig. 1 verdeutlichendes Flussdiagramm.

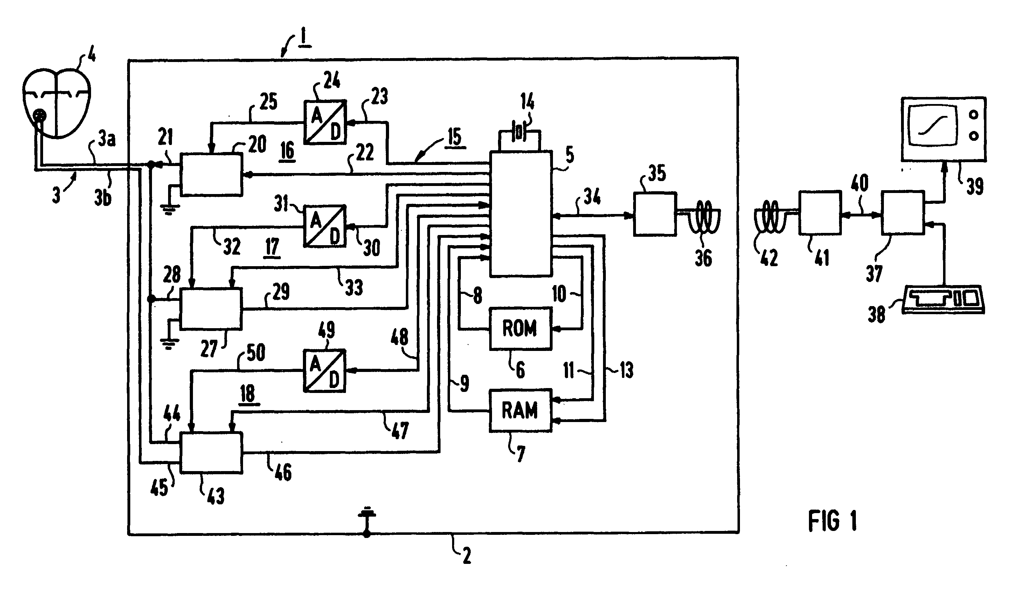

In der Fig. 1 ist die Erfindung anhand eines insgesamt mit (1) bezeichneten Herzschrittmachers dargestellt. Die Bauteile des Herzschrittmachers (1) sind in einem schematisch angedeuteten, hermetisch dichten Gehäuse aus einem elektrisch leitenden Werkstoff, z.B. Titan, aufgenommen. Von dem im VVI-Mode arbeitenden Herzschrittmacher (1) führt eine bipolare Elektrode (3) zu einem schematisch angedeuteten Herz (4) eines Lebewesens und ist dort in ein Ventrikel, vorzugsweise das rechte, eingepflanzt. Die bipolare Elektrode (3) besitzt zwei Leitungen (3a, 3b).

Der Herzschrittmacher (1) umfasst u.a. einen Mikroprozessor (5), dem ein Nur-Lese-Speicher (ROM) (6) und ein Schreib-Lese-Speicher (RAM) (7) zugeordnet sind, die über Datenleitungen (8, 9) und Adressleitungen (10, 11) mit dem Mikroprozessor (5) in Verbindung stehen. Zu dem RAM (7) führt von dem Mikroprozessor (5) ausserdem eine Leitung (13), die zum Umschalten des RAM (7) von Schreib-auf Lesebetrieb und umgekehrt dient. In dem ROM (6) ist ein Programm gespeichert, mittels dessen sämtliche Funktionen des Herzschrittmachers gesteuert werden. Wenn also im folgenden die Rede davon ist, dass der Mikroprozessor (5) eine bestimmte Funktion ausführt, so ist darunter zu verstehen, dass der Mikroprozessor (5) durch Ausführung des im ROM (6) gespeicherten Programms unter Heranziehung von in dem RAM (7) befindlichen Daten und ihm anderweitig, z.B. über eine Eingangs-Beschaltung, zugeführten Daten zur Ausführung der jeweiligen Funktion tätig wird. Wenn die Rede davon ist, dass der Mikroprozessor (5) für einen Parameter einen bestimmten Wert einstellt, so bedeutet dies in der Regel, falls nichts anderes angegeben ist, dass dem bestimmten Wert entsprechende Daten in dem RAM (7) gespeichert sind, auf die der Mikroprozessor (5) zugreifen kann.

Mit dem Mikroprozessor (5) ist ein Quarz (14) verbunden, der zur Erzeugung der für den Betrieb des Mikroprozessors (5) erforderlichen Taktsignale dient und ausserdem die Zeitreferenz für den Betrieb des Herzschrittmachers (1) darstellt.

Der Mikroprozessor (5) des Herzschrittmachers (1) besitzt eine insgesamt mit (15) bezeichnete Eingangs/Ausgangs-Beschaltung, die mehrere Kanäle (16, 17, 18) aufweist.

Der Kanal (16) dient dazu, das Herz (4) erforderlichenfalls mit Stimulationsimpulsen zu versorgen. Der Kanal (16) besitzt daher einen Stimulationsimpuls-Generator (20), der einen mittels einer Ausgangsleitung (21) mit der Leitung (3a) der Elektrode (3) verbundenen Signalausgang und einen ein Bezugspotential führenden Anschluss aufweist. Der das Bezugspotential führende Anschluss ist mit dem Gehäuse (2) elektrisch leitend verbunden. Dies ist dadurch veranschaulicht, dass sowohl der das Bezugspotential führende Anschluss des Stimulationsimpuls-Generators (20) als auch das Gehäuse (2) mit einem Massesymbol versehen sind. Obwohl eine bipolare Elektrode (3) vorhanden ist, erfolgt also die Stimulation unipolar. Der Stimulationsimpuls-Generator (20) ist über eine Leitung (22), die mit einem entsprechenden Ausgang des Mikroprozessors (5) verbunden ist, zur Abgabe eines Stimulationsimpulses aktivierbar. Digitale Daten, die die Dauer und die Amplitude und damit den Energiegehalt der Stimulationsimpulse betreffen, gelangen von dem Mikroprozessor (5) über eine Leitung (23) zu einer Digital/Analog-Schnittstelle (24), die dem Stimulationsimpuls-Generator (20) über eine Steuerleitung (25) den digitalen Daten entsprechende analoge Steuersignale zuführt. Diese stellen den Stimulationsimpuls-Generator (20) derart ein, dass er bei Bedarf Stimulationsimpulse eines bestimmten Energiegehaltes erzeugt und somit das Herz (4) des Lebewesens mit einer bestimmten Stimulationsintensität stimuliert.

Der Kanal (17) besitzt eine erste Detektoreinrichtung (27), die ausschliesslich dazu dient natürliche Herzschläge zu detektieren. Die erste Detektoreinrichtung (27) besitzt einen mit der Leitung (3a) der Elektrode (3) über eine Eingangsleitung (28) verbundenen Signaleingang und einen ein Bezugspotential führenden Anschluss. Dabei handelt sich bei dem Bezugspotential, wie durch das Massesymbol deutlich wird, um das gleiche Bezugspotential, das auch der entsprechende Anschluss des Stimulationsimpuls-Generators (20) und das Gehäuse (2) führen. Obwohl eine bipolare Elektrode (3) vorhanden ist, erfolgt also auch die Detektion natürlicher Herzschläge mittels der ersten Detektoreinrichtung (27) unipolar. Detektiert die erste Detektoreinrichtung (27) in dem ihr über die Leitung (3a) der Elektrode (3) zugeführten, der elektrischen Aktivität des Herzens (4) entsprechenden Signal einen natürlichen Herzschlag, gibt sie über eine Leitung (29) ein dies anzeigendes Signal an einen entsprechenden Eingang des Mikroprozessors (5). Diese Signal gibt die erste Detektoreinrichtung (27) dann ab, wenn in dem der elektrischen Aktivität des Herzens (4) entsprechenden Signal ein Ereignis mit für einen natürlichen Herzschlag typischer Steilheit und/oder Amplitude auftritt. Der Mikroprozessor (5) ist über eine Leitung (30) mit einer Digital/Analog-Schnittstelle (31) verbunden, die ihr von dem Mikroprozessor (5) zugeführte digitale Daten als entsprechende analoge Signale über eine Steuerleitung (32) an die erste Detektoreinrichtung (27) weitergibt. Die digitalen Daten bzw. die entsprechenden analogen Signale dienen dazu, die Empfindlichkeit der ersten Detektoreinrichtung (27), also diejenige Steilheit und/oder Amplitude, einzustellen, die ein Ereignis in dem der elektrischen Aktivität des Herzens (4) entsprechenden Signal wenigstens aufweisen muss, um als natürlicher Herzschlag detektiert zu werden. Über eine Steuerleitung (33) kann der Mikroprozessor ausserdem der ersten Detektoreinrichtung (27) ein Signal zuführen, das diese völlig sperrt, so dass keine auf die Detektion eines natürlichen Herzschlages hinweisenden Signale zu dem Mikroprozessor (5) gelangen können.

Erhält der Mikroprozessor (5) über die Leitung (29) ein der Detektion eines natürlichen Herzschlages mittels der ersten Detektoreinrichtung (27) entsprechendes Signal oder aktiviert er über die Leitung (22) den Stimulationsimpuls-Generator (20) zur Abgabe eines Stimulationsimpulses, beginnt der Mikroprozessor (5) als Zähler zu arbeiten und beginnt eine Anzahl von aus der Schwingung des Quarzes (14) abgeleiteten Taktimpulsen abzuzählen, die einem Basis-Zeitintervall entspricht. Das Basis-Zeitintervall bestimmt diejenige Stimulationsfrequenz, mit der das Herz (4) beim Ausbleiben natürlicher Herzschläge stimuliert wird. Gelangt während des Basis-Zeitintervalls über den Kanal (17) kein Signal zu dem Mikroprozessor (5), das die Detektion eines natürlichen Herzschlags anzeigt, aktiviert der Mikroprozessor (5) bei Ablauf des Basis-Zeitintervalls über die Leitung (22) den Stimulationsimpuls-Generator (20). Im Anschluss an die Abgabe eines Stimulationsimpulses beginnt der Mikroprozessor (5) erneut eine Anzahl von Taktimpulsen abzuzählen, die dem die Stimulationsfrequenz bestimmenden Basis-Zeitintervall entspricht. Erhält der Mikroprozessor (5) dagegen während des Laufs des Basis-Zeitintervalls ein die Detektion eines natürlichen Herzschlags anzeigendes Signal von der ersten Detektoreinrichtung (27), bricht er den beschriebenen Zählvorgang ab, sofern ein weiteres Zeitintervall, die sogenannte Refraktärzeit, abgelaufen ist, und beginnt den beschriebenen Zählvorgang von neuem ohne dass die Abgabe eines Stimulationsimpulses erfolgt. Die Detektion des natürlichen Herzschlages mittels der ersten Detektoreinrichtung (27) inhibiert also die Abgabe eines Stimulationsimpulses. Die Refraktärzeit, die grundsätzlich kürzer ist als das beispielsweise auf Werte zwischen 400 und 2000 Millisekunden programmierbare Basis-Zeitintervall, dauert etwa zwischen 250 und 450 Millisekunden (programmierbar). Die Refraktärzeit unterteilt sich in eine absolute Refraktärzeit mit einer Dauer von gewöhnlich 125 Millisekunden und eine relative Refraktärzeit, auf die die restliche Zeit der gesamten eingestellten Refraktärzeit entfällt. Die Refraktärzeit beginnt jeweils gleichzeitig mit dem Basis-Zeitintervall zu laufen und wird von dem Mikroprozessor (5) im Zuge des gleichen Zählvorgangs ermittelt, der auch zur Ermittlung des Basis-Zeitintervalls dient. Während der absoluten Refraktärzeit ist im Kanal (17) die erste Detektoreinrichtung (27) gesperrt, was dadurch erreicht wird, das der Mikroprozessor (5) der ersten Detektoreinrichtung (27) über die Steuerleitung (33) ein entsprechendes Signal zuführt. Infolge der Sperrung der ersten Detektoreinrichtung (27) ist während der Dauer der absoluten Refraktärzeit mittels der ersten Detektoreinrichtung (27) keinerlei Detektion möglich, d.h. entsprechende Signale können nicht zu dem Mikroprozessor (5) gelangen. Nach Ablauf der absoluten Refraktärzeit aktiviert der Mikroprozessor (5) die erste Detektoreinrichtung (27) wieder, so dass diese in der Lage ist, natürliche Herzschläge zu detektieren. Erfolgt eine Detektion während der relativen Refraktärzeit, bricht der Mikroprozessor (5) im Gegensatz zu einer Detektion nach Ablauf der Refraktärzeit den Zählvorgang zur Ermittlung des Basis-Zeitintervalls nicht ab, sondern führt ihn fort und schliesst ihn mit einer Aktivierung des Stimulationsimpuls-Generators (20) ab. Allerdings setzt der Mikroprozessor (5) nach der Detektion eines natürlichen Herzschlages nochmals die volle Refraktärzeit in Gang. Hierdurch wird erreicht, dass im Falle von Hochfrequenzstörungen, die zu Fehldetektionen führen, unabhängig vom Auftreten natürlicher Herzschläge Stimulationsimpulse mit der durch das jeweils programmierte Basis-Zeitintervall bestimmten Stimulationsfrequenz erzeugt werden. Auch wenn die spontane Herzschlagfrequenz so hoch liegt, dass das Auftreten natürlicher Herzschläge jedesmal innerhalb der relativen Refraktärzeit erfolgt, erfolgt eine Abgabe von Stimulationsimpulsen mit der durch das jeweils programmierte Basis-Zeitintervall bestimmten Stimulationstrequenz, und zwar bis die spontane Herzschlagfrequenz unter eine Frequenz zurückgefallen ist, deren Periodendauer der jeweils eingestellten Refraktärzeit entspricht. Mittels dieser Funktion ist die Beendigung bestimmter Wiedereintrittstachykardien möglich.

Der Mikroprozessor (5) ist über eine Leitung (34) mit einem Telemetrieschaltkreis (35) verbunden, an den eine Sende/Empfangs-Spule (36) angeschlossen ist. Der Herzschrittmacher (1) ist somit in der Lage, mit einem externen Progammiergerät (37) mit einer Tastatur (38) und einem Monitor (39) Daten auszutauschen, da das Programmiergerät (37) über eine Leitung (40) mit einem zweiten Telemetrieschaltkreis (41) verbunden ist, an den wieder eine Sende/Empfangs-Spule (42) angeschlossen ist. Zum Datenaustausch zwischen dem Herzschrittmacher (1) und dem Programmiergerät (37) wird die Sende/Empfangs-Spule (42) des zu dem Programmiergerät (37) gehörigen Telemetrieschaltkreises (41) so auf der Körperoberfläche des den Herzschrittmacher (1) tragenden Lebewesens positioniert, dass sie mit der Sende/Empfangs-Spule (36) des Herzschrittmachers (1) induktiv gekoppelt ist. Es besteht dann die Möglichkeit, dem Programmiergerät (27) die in dem ROM (6) und dem RAM (7) befindlichen Daten zur Überprüfung zuzuführen. Ausserdem besteht die Möglichkeit, dem RAM (7) des Herzschrittmachers (1) von dem Programmiergerät (37) her veränderte bzw. zusätzliche Daten zuzuführen, die das Betriebsverhalten des Herzschrittmachers (1), also dessen Zussamenspiel mit dem zu stimulierenden Herz (4) beeinflussen und verändern. Dieser Vorgang wird gewöhnlich als Programmierung bezeichnet.

Der Kanal (18) der Eingangs/Ausgangs-Beschaltung (15) des Mikroprozessors (5) dient dazu, dem Mikroprozessor (5) Daten zur Verfügung zu stellen, die es diesem anhand des in dem ROM (6) gespeicherten Programmes gestatten, einerseits die Stimulationsintensität, also den Energiegehalt der mittels des Stimulationsimpuls-Generator (20) erzeugten Stimulationsimpulse, so einzustellen dass ein Stimulationsimpuls auch tatsächlich einen stimulierten Herzschlag auslöst, und andererseits die Empfindlichkeit der ersten Detektoreinrichtung (27) so einzustellen, dass eine störungsfreie und sichere Detektion aller natürlicher Herzschläge gewährleistet ist. Der Kanal (18) enthält zu diesem Zweck eine zweite Detektoreinrichtung (43), die sowohl zur Detektion natürlicher als auch stimulierter Herzschläge vorgesehen ist. Die zweite Detektoreinrichtung (43) besitzt zwei Anschlüsse, von denen der eine über die Leitung (44) mit der Leitung (3a) und der andere über eine Leitung (45) mit der Leitung (3b) der bipolaren Elektrode (3) verbunden ist. Die zweite Detektoreinrichtung (43) detektiert im Gegensatz zu der bereits beschriebenen ersten Detektoreinrichtung (27) nicht nur natürliche, sondern wie bereits erwähnt auch stimulierte Herzschläge, also Herzschläge, die auf einen durch den Stimulationsimpuls-Generator (20) abgegebenen Stimulationsimpuls hin auftreten. Detektiert die zweite Detektoreinrichtung (43) einen stimulierten oder einen natürlichen, d.h. spontan auftretenden, Herzschlag, gelangt ein entsprechendes Signal über eine Leitung (46) vom Ausgang der zweiten Detektoreinrichtung (43) zu einem entsprechenden Eingang des Mikroprozessors (5). Der Mikroprozessor (5) ist in der Lage, über eine Steuerleitung (47) durch ein entsprechendes Signal den Ausgang der zweiten Detektoreinrichtung (43) zu sperren. Die Empfindlichkeit der zweiten Detektoreinrichtung (43) ist mittels des Mikroprozessors (5) einstellbar, indem dieser über die Leitung (48) einer Digital/Analog-Schnittstelle (49) digitale Daten zuführt, die die Digital/Analog-Schnittstelle (49) in ein entsprechendes analoges Signal umsetzt, das der zweiten Detektoreinrichtung (43) über eine Steuerleitung (50) zugeführt ist. Das analoge Signal bestimmt in noch näher zu erläuternder Weise die Empfindlichkeit der zweiten Detektoreinrichtung, die der Mikroprozessor (5) normalerweise höher als die Empfindlichkeit der ersten Detektoreinrichtung (27), wenigstens jedoch gleich hoch einstellt.

Anders als im Falle der ersten Detektoreinrichtung (27), deren Ausgang der Mikroprozessor (5) nur nach Ablauf der absoluten Refraktärzeit freischaltet, müssen im Falle der zweiten Detektoreinrichtung (43) Detektionen auch während der absoluten Refraktärzeit erfolgen können, um die Detektion stimulierter Herzschläge zu ermöglichen. Der Mikroprozessor (5) schaltet also den Ausgang der zweiten Detektoreinrichtung (43) nicht nur nach Ablauf der absoluten Refraktärzeit bis zum Auftreten eines natürlichen Herzschlages oder der Abgabe eines Stimulationsimpulses zur Detektion natürlicher Herzschläge frei, sondern ausserdem einige Millisekunden nach der Abgabe eines Stimulationsimpulses für ein kurzes Zeitintervall, z.B. 100 Millisekunden, zur Detektion stimulierter Herzschläge frei. Dabei ist jedoch wesentlich, dass weder die Detektion eines stimulierten Herzschlages noch die Detektion eines natürlichen Herzschlages mittels der zweiten Detektoreinrichtung (43) die Abgabe eines Stimulationsimpulses inhibieren kann. Dies kann nur durch die Detektion eines natürlichen Herzschlages mittels der ersten Detektoreinrichtung (27) unter den zuvor beschriebenen Voraussetzungen erfolgen.

Die selbsttätige Einstellung des Energiegehaltes der mittels des Stimulationsimpuls-Generators (20) erzeugten Stimulationsimpulse erfolgt derart, dass der Mikroprozessor (5) nach Abgabe eines Stimulationsimpulses prüft, ob von dem dann freigeschalteten Ausgang der zweiten Detektoreinrichtung (43) über die Leitung (46) ein die Detektion eines stimulierten Herzschlages anzeigendes Signal ankommt. Ist dies nicht der Fall, erhöht der Mikroprozessor (5) den Energiegehalt des nächsten Stimulationsimpulses, indem er der Digital/Analog-Schnittstelle (24) digitale Daten zuführt, die diese in ein analoges Signal umsetzt, das den Stimulationsimpuls-Generator (20) derart einstellt, dass dieser einen Stimulationsimpuls mit einem gegenüber dem zuvor vorhandenen Energiegehalt erhöhten Energiegehalt abgibt. Dies erfolgt solange, bis eine Einstellung für den Energiegehalt der Stimulationsimpulse gefunden ist, bei der die zweite Detektoreinrichtung (43) nach einem Stimulationsimpuls jeweils einen stimulierten Herzschlag detektiert. Dabei stellt der Mikroprozessor (5) über die Digital/Analog-Schnittstelle (24) den Stimulationsimpuls-Generator (20) derart ein, dass der Energiegehalt der erzeugten Stimulationsimpulse der Summe aus einem Mindest-Energiegehalt (Mindestwert des Energiegehaltes), bei dem die zweite Detektoreinrichtung (43) gerade noch nach der Abgabe eines Stimulationsimpulses einen stimulierten Herzschlag detektiert, und einem Sicherheitsmarginal, z.B. 50 % des Mindest-Energiegehaltes, entspricht. Dabei entspricht der Mindest-Energiegehalt der sogenannten Reizschwelle, die der Energiegehalt eines Stimulationsimpulses wenigstens erreichen muss, um einen stimulierten Herzschlag auslösen zu können.

Zur Ermittlung des Mindest-Energiegehaltes senkt der Mikroprozessor (5) den Energiegehalt der Stimulationsimpulse ausgehend von einem Wert, bei dem die zweite Detektoreinrichtung (43) nach jedem Stimulationsimpuls einer Folge von Stimulationsimpulsen einen stimulierten Herzschlag detektiert, allmählich so weit ab, bis wenigstens nach einzelnen Stimulationsimpulsen mittels der zweiten Detektoreinrichtung (43) kein stimulierter Herzschlag mehr detektierbar ist. Ausgehend von dem so gefundenen Wert des Energiegehaltes erhöht der Mikroprozessor (5) den Energiegehalt der Stimulationsimpulse allmählich wieder, und zwar gerade so weit, bis die zweite Detektoreinrichtung (42) nach jedem Stimulationsimpuls wieder einen stimulierten Herzschlag detektiert. Der so gefundene Wert stellt den Mindest-Energiegehalt der Stimulationsimoulse dar.

Durch die beschriebene Einstellung des Energiegehaltes der Stimulationsimpulse wird erreicht, dass einerseits die Sicherheit des Patienten gewährleistet ist, da die Stimulation stets mit Stimulationsimpulsen erfolgt, deren Energiegehalt um ein Sicherheitsmarginal oberhalb des Mindest-Energiegehaltes liegt. Andererseit ist sichergestellt, dass der Energiebedarf des Gerätes durch die Abgabe der Stimulationsimpulse nicht höher als nötig ist, da sich der Energiegehalt der Stimulationsimpulse stets an dem erforderlichen Mindest-Energiegehalts orientiert.

Die selbsttätige Einstellung der Empfindlichkeit der ersten Detektoreinrichtung (27) erfolgt derart, dass der Mikroprozessor (5) nach Ablauf der absoluten Refraktärzeit prüft, ob ein mittels der zweiten Detektoreinrichtung (43) detektierter natürlichen Herzschlag auch mittels der ersten Detektoreinrichtung (27) detektiert wurde. Dies ist möglich, da der Mikroprozessor (5) jeweils nach dem Ablauf der absoluten Refraktärzeit die erste und die zweite Detektoreinrichtung (27 bzw. 43) aktiviert. Erfolgt die Detektion eines natürlichen Herzschlages nur mittels der zweiten Detektoreinrichtung (43) erhöht der Mikroprozessor (5) die Empfindlichkeit der ersten Detektoreinrichtung (27), indem er der Digital/Analog-Schnittstelle (31) digitale Daten zuführt, die diese in ein analoges Signal umwandelt, das die Empfindlichkeit der ersten Detektoreinrichtung (27) derart einstellt, dass diese gegenüber dem zuvor eingestellten Wert erhöht ist. Dies erfolgt solange, bis eine Einstellung für die Empfindlichkeit der ersten Detektoreinrichtung (27) gefunden ist, bei der die erste Detektoreinrichtung (27) mittels der zweiten Detektoreinrichtung (43) detektierte natürliche Herzschläge ebenfalls detektiert. Es versteht sich, dass für diesen Vorgang die Empfindlichkeit der zweiten Detektoreinrichtung (43) wenigstens der jeweils eingestellten Empfindlichkeit der ersten Detektoreinrichtung (27) entsprechen muss. Zweckmässigerweise stellt jedoch der Mikroprozessor (5) mittels der Digital/Analog-Schnittstelle (49) die Empfindlichkeit der zweiten Detektoreinrichtung (43) deutlich höher als die Empfindlichkeit der ersten Detektoreinrichtung (27) ein. Dabei ist jedoch zu beachten, dass die Empfindlichkeit der zweiten Detektoreinrichtung (43) nicht beliebig erhöht werden darf, da sichergestellt sein muss, dass Störungen und Rauschen nicht zu Fehldetektionen führen. Dabei stellt der Mikroprozessor (5) über die Digital/Analog-Schnittstelle (31) die Empfindlichkeit der ersten Detektoreinrichtung (27) derart ein, dass sie um ein Sicherheits-Marginal der Empfindlichkeit höher als eine Mindest-Empfindlichkeit (Mindestwert der Empfindlichkeit) ist, bei der die erste Detektoreinrichtung (27) gerade noch einen mittels der zweiten Detektoreinrichtung (43) detektierten natürlichen Herzschlag ebenfalls detektiert. Als Sicherheits-Marginal kommen z.B. 50 % der Mindest-Empfindlichkeit in Frage.

Zur Ermittlung der Mindest-Empfindlichkeit senkt der Mikroprozessor (5) die Empfindlichkeit der ersten Detektoreinrichtung (27) ausgehend von einem Wert, bei dem die erste Detektoreinrichtung (27) jeden mittels der zweiten Detektoreinrichtung (43) detektierten natürlichen Herzschlag ebenfalls detektiert, allmählich so weit ab, bis wenigstens einzelne mittels der zweiten Detektoreinrichtung (43) detektierte natürliche Herzschläge mittels der ersten Detektoreinrichtung (27) nicht mehr detektierbar sind. Ausgehend von dem so gefundenen Wert der Empfindlichkeit erhöht der Mikroprozessor (5) die Empfindlichkeit der ersten Detektoreinrichtung (27) allmählich wieder, und zwar gerade so weit, bis die erste Detektoreinrichtung (27) jeden mittels der zweiten Detektoreinrichtung (43) detektierten natürlichen Herzschlag ebenfalls detektiert. Bei dem so gefundenen Wert der Empfindlichkeit der ersten Detektoreinrichtung (27) handelt es sich um die Mindest-Empfindlichkeit.

Die beschriebene Einstellung der Empfindlichkeit der ersten Detektoreinrichtung (27) bietet den Vorteil, dass einerseits im Interesse des Patienten durch das Sicherheits-Marginal der Empfindlichkeit gewährleistet ist, dass alle natürlichen Herzschläge mittels der ersten Detektoreinrichtung (27) tatsächlich detektiert werden können. Andererseits ist infolge des Umstandes, dass die Einstellung der Empfindlichkeit auf Grundlage der jeweiligen Mindest-Empfindlichkeit erfolgt, sichergestellt, dass die tatsächlich vorhandene Empfindlichkeit zu keinem Zeitpunkt höher ist, als dies im Interesse des Patienten unbedingt erforderlich ist, so dass die Gefahr von Fehldetektionen nur sehr gering ist.

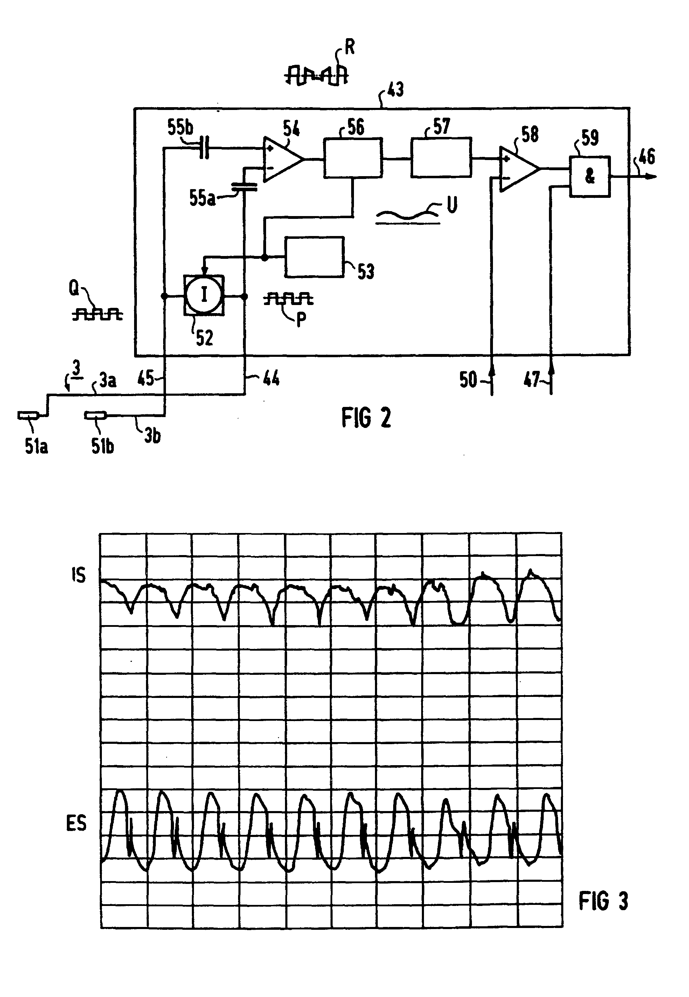

Ein Ausführungsbeispiel für die in Fig. 1 nur schematisch angedeutete zweite Detektoreinrichtung (43) ist in Fig. 2 dargestellt. Es handelt sich hier um eine Detektoreinrichtung, die ein Signal bildet, das der zwischen ihren Anschlüssen vorhandenen äusseren Impedanz entspricht. Dabei soll der Begriff äussere Impedanz zum Ausdruck bringen, dass in der äusseren Impedanz die Eingangsimpedanz der zweiten Detektoreinrichtung (43) nicht enthalten ist. Bei der äusseren Impedanz handelt es sich also sozusagen um die Quellimpedanz, die die zweite Detektoreinrichtung (43) sieht. Diese Quellimpedanz ergibt sich aus der Impedanz der in Fig. 2 schematisch angedeuteten bipolaren Elektrode (3) mit ihren Leitungen (3a und 3b) und der Impedanz des zwischen den bei implantierte Elektrode mit dem Herz in elektrisch leitender Verbindung stehenden Kontaktteilen (51a and 51b) befindlichen Herzmuskelgewebes.

Infolge der Herzaktion ändert sich die Impedanz des Herzmuskelgewebes, und zwar im Einklang mit der Herzaktion. Dies ist aus der Fig. 3 ersichtlich, in der für eine Anzahl natürlicher Herzschläge das der elektrischen Aktivität des Herzens entsprechende elektrische Signal, das mit ES bezeichnet ist, im Vergleich zu einem der elektrischen Impedanz der Herzens entsprechenden Signal, das mit IS bezeichnet ist, dargestellt ist.

Das der impedanz des Herzens und damit der Herzaktion entsprechende elektrische Signal wird im Falle der zweiten Detektoreinrichtung (43) dadurch erhalten, dass diese eine bipolare modulierbare Stromquelle (52) aufweist, die parallell zu der Quellimpedanz zwischen die Anschlüsse der zweiten Detektoreinrichtung (43) geschaltet ist. Die Stromquelle (52) ist mit einem Oszillator (53) verbunden, der ein bei P angedeutetes Rechtecksignal mit einer konstanten Amplitude und mit einer konstanten Frequenz erzeugt, die deutlich grösser als die zu erwartender Herzschlagfrequenz ist. Als Frequenz des Rechtecksignals kommen z.B. 4 kHz infrage.

Der Oszillator (53) moduliert die Stromquelle (52) derart, dass diese einen Wechselstrom konstanter Amplitude erzeugt, der hinsichtlich seine Frequenz und Kurvenform dem Signal des Oszillators (53) entspricht und zur Nullinie symmetrisch ist. Dieser Wechselstrom, der bei Q schematisch angedeutet ist, fliesst durch die an die zweite Detektoreinrichtung (43) angeschlossene Quellimpedanz.

Der über der Quellimpedanz infolge des Wechselstroms entstehende Spannungsabfall wird mittels eines Differenzverstärkers (54) verstärkt, dessen Eingänge über Koppelkondensatoren (55a und 55b) mit den Leitungen (44 und 45) verbunden sind. Infolge der Koppelkondensatoren (55a, 55b) verstärkt der Differenzverstärker (54) nur den durch den Wechselstrom hervorgerufenen Spannungsabfall, der ein Mass für die jeweils vorhandene Quellimpedanz darstellt. Da sich die Impedanz der Elektrode (3) nicht ändert, erfolgt die Änderung der Quellimpedanz ausschliesslich auf Grund der Impedanzänderungen des Herzens infolge der Herzaktion. Das Ausgangssignal des Differenzverstärkers (54), das bei R angedeutet ist und ebenfalls symmetrisch zur Nullinie verläuft, spiegelt also den Impedanzverlauf des Herzens und damit die Herzaktion wieder.

Das Ausgangssignal des Differenzverstärkers (54) ist einem Demodulator (56) zugeführt. Dieser demoduliert das Ausgangssignal des Differenzverstärkers (54) infolge des Umstandes, dass ihm das Signal des Oszillators (53) zugeführt ist, in korrekter Phasenbeziehung zu der Modulation der Stromquelle (52), so dass am Ausgang des Demodulators (56) ein bei U angedeutetes, in seinem Verlauf dem Impedanzverlauf des Herzens bzw. der Herzaktion entsprechendes unipolares Signal vorliegt, das im wesentlichen dem Signal IS gemäss FIG 3 entspricht.

Dieses Signal ist einem Bandpassfilter (57) zugeführt, dessen Übertragungsfunktion derart gewählt ist, das nur diejenigen Signalanteile, die hinsichtliche ihr Frequenz bzw. Steilheit für Herzschläge, seien es natürliche oder stimulierte, typisch sind, das Bandpassfilter (57) passieren können.

Das Ausgangssignal des Bandpassfilters (57) gelangt zu dem einen Eingang eines Komparators (58), der dessen Amplitude mit einem Schwellwertsignals vergleicht, das seinem anderen Eingang über die Steuerleitung (50) zugeführt ist. Bei dem Schwellwertsignal handelt es sich um das Ausgangssignal der Digital/Analog-Schnittstelle (49). Überschreitet die Amplitude des Ausgangssignals des Bandpassfilters (57) den Pegel des Schwellwertsignals, springt das Ausgangssignal des Komparators (58) von seinem einen Extremwert auf seinen anderen Extremwert um, wobei bezüglich des Ausgangssignals des Komparators (58) der Ursprungzustand wieder hergestellt wird, wenn die Amplitude des Ausgangssignals des Bandpassfilters (57) unter das Schwellwertsignal fällt. Das Ausgangssignals des Komparators (58) besitzt den Wert logisch "0", solange die Amplitude des Ausgangssignals des Bandpassfilters (57) das Schwellwertsignal nicht überschreitet. Tritt eine Überschreitung des Schwellwertsignals auf, springt das Ausgangssignal des Komparators (58) auf logisch "1" um. Wenn also der Mikroprozessor (5) über die Digital/Analog-Schnittstelle (49) das Schwellwertsignals derart einstellt, dass es einem Pegel entspricht, die das bei natürlichen wie auch stimulierten Herzschlägen auftretende Ausgangssignal des Bandpassfilter (57) überschreitet, steht am Ausgang des Komparators (58) ein Signal zur Verfügung, das im Falle der Detektion eines Herzschlages den Pegel logisch "1" aufweist.

Das Ausgangssignal des Komparators (58) wird jedoch dem Mikroprozessor (5) nicht direkt zugeführt, sondern gelangt zu dem einen Eingang eines UND-Gliedes (59), dessen Ausgang mit der zu dem Mikroprozessor (5) führenden Leitung (46) verbunden ist. Mit dem anderen Eingang des UND-Gliedes (59) ist die Steuerleitung (47) verbunden. Es wird somit deutlich, dass nur dann die Detektion eines natürlichen oder stimulierten Herzschlages anzeigende Signale von dem Komparator (58) über die Leitung (46) zu dem Mikroprozessor (5) gelangen können, wenn dieser den Ausgang der zweiten Detektoreinrichtung (43) freischaltet, indem er dem UND-Glied (59) über die Steuerleitung (47) ein Signal mit dem Pegel logisch "1" zuführt, was nur während der im Zusammenhang mit der Fig. 1 erläuterten Zeitintervalle der Fall ist.

Es besteht ausserdem in in Fig. 2 nicht dargestellter Weise die Möglichkeit, die gesamte Detektoreinrichtung (43) während solcher Zeiträume, in denen sie nicht benötigt wird, stromlos zu schalten, um den Strombedarf des Herzsehrittmachers zu senken.

Der Oszillator (53) muss nicht unbedingt als besondere Schaltung vorhanden sein. Das zur Modulation der Stromquelle (52) und zur Synchronisation des Demodulators (56) benötigte Rechtecksignal kann auch aus der Schwingung des mit dem Mirkoprozessor (5) verbundenen Quarzes (14) abgeleitet werden.

Die beschriebene Ausbildung der zweiten Detektoreinrichtung (43) bietet im Zusammenhang mit der Detektion stimulierter Herzschläge den Vorteil, dass das der Impedanz des Herzens entsprechende elektrische Signal ausschliesslich auf Grundlage desjenigen Spannungsabfalls über der Quellimpedanz ermittelt wird, der infolge des von der Stromquelle (52) abgegebenen Wechselstromes auftritt. Nachteilige Auswirkungen infolge des Umstandes, das unmittelbar nach einem Stimulationsimpuls, und zu dieser Zeit muss die Detektion eines stimulierten Herzschlages erfolgen, das Herzmuskelgewebe im Bereich des Kontaktes (51a) der Elektrode (3) ein von dem Potential des Herzmuskelgewebes im Bereich des Kontaktes (51b) abweichendes Potential aufweist, können also nicht auftreten.

Im Zusammenhang mit der selbsttätigen Einstellung der Empfindlichkeit der ersten Detektoreinrichtung (27) bietet die beschriebene Ausführung der zweiten Detektoreinrichtung (43) den Vorteil, dass die Detektion ein und desselben natürlichen Herzschlages mittels der ersten und der zweiten Detektoreinrichtung (27 bzw. 43) nicht gleichzeitig erfolgt, was bei der Verarbeitung der entsprechenden Signale mittels des Mikroprozessors (5) zu Problemen führen könnte. Vielmehr erfolgt die Detektion mittels der zweiten Detektoreinrichtung (43) kurze Zeit nach der entsprechenden Detektion mittels der ersten Detektoreinrichtung (27). Im Zusammenhang mit der selbsttätigen Einstellung der Empfindlichkeit der ersten Detektoreinrichtung (27) muss der Mikroprozessor (5) also nur überprüfen, ob der Detektion eines natürlichen Herzschlages mittels der zweiten Detektoreinrichtung (43) eine entsprechende Detektion mittels der ersten Detektoreinrichtung (27) vorangegangen ist. Störungen der ersten Detektoreinrichtung (27) durch den Wechselstrom der zweiten Detektoreinrichtung (43) sind nicht zu befürchten, da die Frequenz des Wechselstromes so hoch liegt, dass die erste Detektoreinrichtung darauf nicht ansprechen kann.

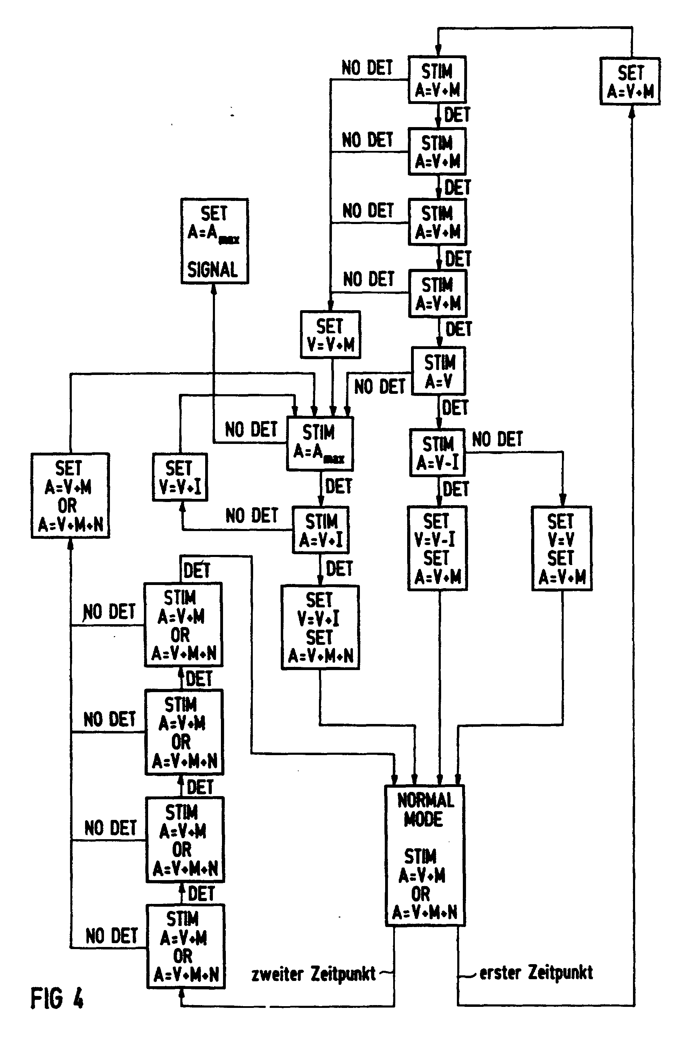

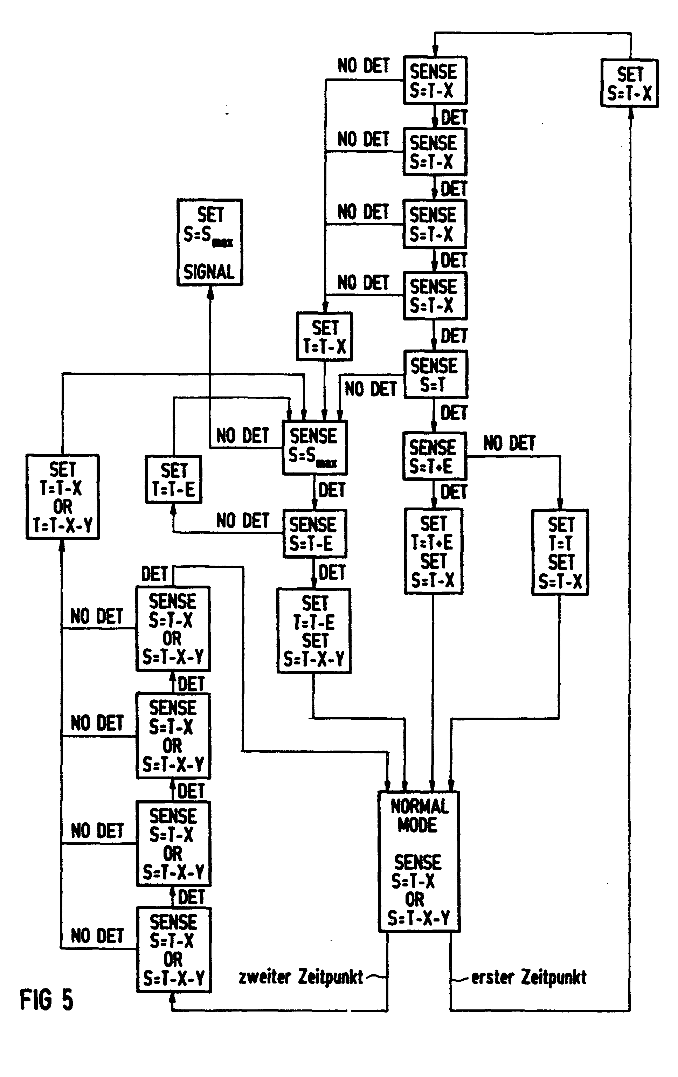

Nachdem im Zusammenhang mit der Fig. 1 bereits beschrieben wurde, wie die selbsttätige Einstellung der Stimulationsintensität und die Einstellung der Empfindlichkeit erfolgen können, werden diesbezügliche Betriebsverfahren im Zusammenhang mit den Figuren 4 und 5 näher erläutert.

Die Fig. 4 verdeutlicht das Betriebsverfahren zur selbsttätigen Einstellung der Stimulationsintensität, also des Energiegehaltes der Stimulationsimpulse. Demnach findet keine kontinuierliche Einstellung der Stimulationsintensität statt. Vielmehr befindet sich der Herzschrittmacher die meiste Zeit in einer mit "NORMAL MODE" bezeichneten Betriebsweise, in der die zweite Detektoreinrichtung (43) stromlos geschaltet ist und der Mikroprozessor (5) in keiner Weise zur Einstellung des Energiegehalts der Stimulationsimpulse tätig wird. Dies geschieht aus Gründen der Energieersparnis und ist ohne Nachteil für den Patienten möglich, da sich die Reizschwelle in der Regel nur sehr langsam ändert, so dass es genügt, eine selbsttätige Einstellung des Energiegehaltes der Stimulationsimpulse in grösseren Zeitabständen vorzunehmen. Im vorliegenden Falle erfolgt dies zu ersten Zeitpunkten, zwischen denen jeweils ein Zeitintervall in der Grössenordnung von Stunden liegt. Während dieser Zeitintervalle stimuliert der Stimulationsimpuls-Generator (20) das Herz mit Stimulationsimpulsen desjenigen Energiegehaltes A, der bei der zuletzt vorgenommenen selbsttätigen Einstellung des Energiegehaltes eingestellt wurde. Dieser Energiegehalt A setzt sich zusammen aus dem der Reizschwelle entsprechenden Mindest- Energiegehalt V, der erforderlich ist, um einen mittels der zweiten Detektoreinrichtung (43) detektierbaren stimulierten Herzschlag auszulösen, und dem mit M bezeichneten Sicherheits-Marginal. In besonderen Fällen, die noch erläutert werden, kann noch ein zusätzliches Sicherheits-Marginal N, das z.B. 25 % des Mindest-Energiegehalts V betragen kann, vorhanden sein.

Dass der Stimulationsimpuls-Generator (20) im "NORMAL MODE" mit dem erwähnten Energiegehalt (A) stimuliert, ist durch die Angabe STIM A = V+M bzw. STIM A = V+M+N verdeutlicht. In der Fig. 4 bedeutet die Angabe STIM verbunden mit der Angabe eines bestimmten Energiegehaltes A übrigens immer, dass ein Stimulationsimpuls des jeweiligen Energiegehaltes A abgegeben wird.

Im Zuge der selbsttätigen Einstellung der Stimulationsintensität, die zu jedem ersten Zeitpunkt erfolgt, wird die zweite Detektoreinrichtung aktiviert und zunächst für eine definierte Anzahl von Stimulationsimpulsen, in Fig. 4 sind es beispielhaft vier Stimulationsimpulse mit dem Energiegehalt A = V+M, überprüft, ob die zweite Detektoreinrichtung (43) jeweils einen stimulierten Herzschlag detektiert. Die Detektion eines stimulierten Herzschlages ist in Fig. 4 durch die Angabe DET veranschaulicht während das Ausbleiben der Detektion eines stimulierten Herzschlages nach einer Stimulation mit der Angabe NO DET verdeutlicht ist. Der Energiegehalt A der Stimulationsimpulse beträgt dabei übrigens unabhängig davon, ob zuvor das zusätzliches Sicherheits-Marginal N vorhanden war, A = V + M.

Wird für jeden der definierten Anzahl von Stimulationsimpulsen ein nachfolgender stimulierter Herzschlag detektiert, besitzt der nächste Stimulationsimpuls den zuletzt gefundenen Mindest-Energiegehalt V. Führt auch dieser Stimulationsimpuls zur Detektion eines stimulierten Herzschlages, wird bei der nachfolgenden Stimulation ein Stimulationsimpuls mit einem nochmals um einen definierten Schritt I verringerten Energiegehalt A = V-I abgegeben. Der Schritt I kann z.B. ein bestimmter Bruchteil des maximal möglichen Energiegehaltes Aₘₐₓ der Stimulationsimpulse sein. Führt auch dieser Stimulationsimpuls zur Detektion eines stimulierten Herzschlages, bedeutet dies, dass die Reizschwelle abgesunken ist. Als neuer Mindest-Energiegehalt V wird daher der zuletzt vorhandene Wert vermindert um den Schritt I gesetzt und als neuer Energiegehalt A der Stimulationsimpulse der neue Mindest-Energiegehalt V erhöht um das Sicherheits-Marginal M gesetzt. Dies wird in Fig. 4 durch die Angaben SET V = V-I und SET A = V+M. Im übrigen bedeutet in Fig. 4 die Angabe SET in Verbindung mit der Angabe eines Parameters immer, dass diesem Parameter ein neuer Wert zugeordnet wird. Mit dem neu gesetzten Energiegehalt A, der wohlgemerkt um den Schritt I geringer ist, als der zuvor vorhandene, stimuliert der Herzschrittmacher dann im "NORMAL MODE".

Es wäre zwar grundsätzlich denkbar, dass die Reizschwelle zwischen zwei aufeinanderfolgenden ersten Zeitpunkten so weit absinkt, dass eine Absenkung des Mindest-Energiegehaltes V um mehr als einen Schritt I möglich wäre. Dennoch wird im Interesse der Sicherheit des Patienten nur um einen Schritt I abgesenkt. Sollte sich die Reizschwelle tatsächlich auf einem Niveau stabilisieren, das eine weitere Absenkung des Energiegehaltes A gestattet, so erfolgt die weitere Absenkung im Zuge der nächsten selbsttätigen Einstellung(en) des Energiegehaltes A noch frühzeitig genug.

Führt der Stimulationsimpuls mit dem Energiegehalt A = V-I nicht zur Detektion eines stimulierten Herzschlages, bedeutet dies, dass sich die Reizschwelle seit dem letzten ersten Zeitpunkt nicht verändert hat. Daher werden wieder der alte Mindest-Energiegehalt V und der alte Energiegehalt A gesetzt, bevor die Rückkehr in den "NORMAL MODE" erfolgt.

Führt bereits die Stimulation mit dem zuletzt gefundenen Mindest-Energiegehalt V nicht zur Detektion eines stimulierten Herzschlages, ist also die Reizschwelle angestiegen, wird zunächst ein Stimulationsimpuls mit dem maximal möglichen Energiegehalt Aₘₐₓ abgegeben, um sicherzustellen, dass nicht mehr als ein Stimulationsimpuls erfolglos bleibt.

Wird nach diesem Stimulationsimpuls mit dem maximalen Energiegehalt Aₘₐₓ ein stimulierter Herzschlag detektiert, erfolgt als nächstes eine Stimulation mit einem Energiegehalt A = V+I, der dem letzten Mindest-Energiegehalt V erhöht um einen Schritt I entspricht. Führt dies zur Detektion eines stimulierten Herzschlages, wird der neue Mindest-Energiegehalt V entsprechend festgesetzt. Als neuer Energiegehalt A wird jedoch anders als zuvor nicht nur die Summe aus dem neuen Mindest-Energiegehalt V und dem Sicherheits-Marginal M eingestellt. Vielmehr erfolgt noch eine Erhöhung des Energiegehaltes A um das bereits erwähnte zusätzliche Sicherheits-Marginal N. Dadurch wird bis zum nächsten ersten Zeitpunkt, d.h. bis zur nächsten selbsttätigen Einstellung des Energiegehaltes A, im "NORMAL MODE" mit einem Energiegehalt A = V+M+N stimuliert. Dies geschieht, um für den Fall eines weiteren Ansteigens der Reizschwelle im Interesse der Sicherheit des Patienten bereits entsprechende Vorkehrungen zu treffen. Für den Fall, dass bis zum nächsten ersten Zeitpunkt kein weiterer Anstieg der Reizschwelle auftritt, entfällt, wie sich aus den vorstehenden Ausführungen ergibt, dass zusätzliche Sicherheits-Marginal N wieder.

Führt die Stimulation mit dem um einen Schritt I erhöhten Mindest-Energiegehalt V nicht zur Detektion eines natürlichen Herzschlages, wird der Mindest-Energiegehalt V um einen weiteren Schritt I erhöht. Vor einer Stimulation mit dem nochmals erhöhten Mindest-Energiegehalt V erfolgt aber zunächst eine Stimulation mit dem maximalen Energiegehalt Aₘₐₓ. Erst wenn danach ein stimulierter Herzschlag detektiert wird, erfolgt die Stimulation mit dem nochmals erhöhten Mindest-Energiegehalt V. Dies wiederholt sich solange, bis ein Mindest-Energiegehalt V gefunden ist, der zur Detektion eines stimulierten Herzschlages führt, worauf der neue Energiegehalt A für den "NORMAL MODE" wie unmittelbar vorstehend beschrieben eingestellt wird, der entsprechend der Anzahl von Schritten I und um das zusätzliche Sicherheits-Marginal N höher ist als der vorher vorhandene Energiegehalt A.

Führt auch eine Stimulation mit dem maximalen Energiegehalt Aₘₐₓ nicht zur Detektion eines stimulierten Herzschlages, wird als Energiegehalt A für alle weiteren Stimulationen der maximale Energiegehalt Aₘₐₓ eingestellt und die zweite Detektoreinrichtung (43) stromlos geschaltet. Ausserdem werden, dies ist durch die Angabe SIGNAL versinnbildlicht, Massnahmen getroffen, die bei der nächsten Kommunikation des Programmers (37) mit dem Herzschrittmacher (1) dazu führen, dass eine Meldung ausgegeben wird, durch die der behandelnde Arzt erkennen kann, dass eine selbsttätige Einstellung des Energiegehaltes A der Stimulationsimpulse nicht möglich war und deshalb auf den maximalen Energiegehalt Aₘₐₓ übergegangen wurde.

Führt bereits einer der definierten Anzahl von Stimulationsimpulsen, die wie beschrieben zu Beginn der selbsttätigen Einstellung des Energiegehaltes A der Stimulationsimpulse abgegeben werden, nicht zur Detektion eines stimulierten Herzschlages, bedeutet dies, dass die Reizschwelle, z.B. durch eine Verlagerung des in das Herz eingepflanzten Endes der Elektrode, so weit angestiegen ist, dass ein Mindest-Energiegehalt V der Stimulationsimpulse erforderlich ist, der grösser ist als der Energiegehalt A = V+M. Es wird daher zunächst der Mindest-Energiegehalt V auf V = V+M erhöht, bevor die Abgabe eines Stimulationsimpulses mit maximalem Energiegehalt Aₘₐₓ erfolgt, an die sich die zuvor beschriebenen Verfahrensschritte zur Ermittlung eines neuen Mindest-Energiegehalts V und die Festsetzung des neuen Energiegehaltes A unter Berücksichtigung des zusätzlichen Sicherheits-Marginals N anschliessen, bevor die Rückkehr in den "NORMAL MODE" erfolgt.

Zwischen zwei aufeinanderfolgenden ersten Zeitpunkten wird wenigstens zu einem zweiten Zeitpunkt, sind mehrere zweite Zeitpunkte vorgesehen, sind diese jeweils durch ein Zeitintervall mit einer Dauer in der Grössenordnung von beispielsweise Minuten voneinander getrennt, geprüft, ob nach einer definierten Anzahl von Stimulationsimpulsen mit dem zuletzt eingestellten Energiegehalt, also A = V+M oder A = V+M+N, jeweils die Detektion eines stimulierten Herzschlages erfolgt. Zu diesem Zweck wird der "NORMAL MODE" verlassen und die zweite Detektoreinrichtung (43) aktiviert. Wird für alle Stimulationsimpulse der definierten Anzahl, im Falle der Fig. 4 sind dies vier, das Auftreten eines stimulierten Herzschlages detektiert, erfolgt die Rückkehr in den "NORMAL MODE". Bleibt dagegen für einen der Stimulationsimpulse die Detektion eines stimulierten Herzschlages aus, erfolgt als nächstes eine Stimulation mit dem maximalen Energiegehalt Aₘₐₓ. Im Anschluss daran wird in der bereits beschriebenen Weise ein neuer Mindest-Energiegehalt V und ein neuer Energiegehalt A = V+M+N der Stimulationsimpulse festgesetzt und mit diesem Wert in den "NORMAL MODE" zurückgekehrt.

Die beschriebene Kontrolle zu den zweiten Zeitpunkten erfolgt ebenfalls im Interesse der Sicherheit des Patienten, um etwaigen Störungen oder abnormalen Änderungen der Reizschwelle rasch Rechnung tragen zu können.

Wie ein Vergleich der Figuren 4 und 5 zeigt, ähnelt das in Fig. 5 dargestellte Betriebserfahren zur selbsttätigen Einstellung der Empfindlichkeit der ersten Detektoreinrichtung (27) dem Betriebsverfahren zur selbsttätigen Einstellung des Energiegehaltes der Stimulationsimpulse.

Auch bezüglich der Empfindlichkeit der ersten Detektoreinrichtung (27) findet also keine kontinuierliche Einstellung statt. Vielmehr befindet sich der Herzschrittmacher die meiste Zeit in seiner mit "NORMAL MODE" bezeichneten Betriebsweise, in der die zweite Detektoreinrichtung (43) stromlos geschaltet ist. Dies ist auch im Hinblick auf die Einstellung der Empfindlichkeit der ersten Detektoreinrichtung (27) ohne Nachteil für den Patienten möglich, da sich die Verhältnisse in der Regel nicht so rasch ändern, dass eine kontinuierliche Einstellung der Empfindlichkeit der ersten Detektoreinrichtung (27) erforderlich wäre. Es genügt also eine Einstellung der Empfindlichkeit zu ersten Zeitpunkten vorzunehmen, zwischen denen jeweils ein Zeitintervall mit einer Dauer in der Grössenordnung von Stunden liegt. Die ersten Zeitpunkte zur selbsttätigen Einstellung der Empfindlichkeit der ersten Detektoreinrichtung (27) sind zweckmässigerweise so gewählt, dass sie unmittelbar vor oder unmittelbar nach den ersten Zeitpunkten liegen, zu denen die selbsttätige Einstellung des Energiegehaltes der Stimulationsimpulse erfolgt.

Während der zwischen aufeinanderfolgenden ersten Zeitpunkten liegenden Zeitintervalle entspricht die Empfindlichkeit der ersten Detektoreinrichtung (27) der bei der zuletzt vorgenommenen selbsttätigen Einstellung eingestellten Empfindlichkeit. Diese Empfindlichkeit S setzt sich zusammen aus der Mindest-Empfindlichkeit T, die erforderlich ist, um einen mittels der zweiten Detektoreinrichtung (43) detektiereten natürlichen Herzschlag auch mit der ersten Detektoreinrichtung (27) detektieren zu können, und dem mit X bezeichneten Sicherheits-Marginal der Empfindlichkeit. In besonderen Fällen, die noch erläutert werden, kann noch ein zusätzliches Sicherheits-Marginal der Empfindlichkeit Y, das z.B. 25 % der Mindest-Empfindlichkeit T betragen kann, vorhanden sein.

Dass die erste Detektoreinrichtung (27) auf die erwähnte Empfindlichkeit S eingestellt ist, ist durch die Angabe SENSE S = T-X bzw. SENSE S = T-X-Y verdeutlicht.

Dabei werden die Sicherheits-Marginale X bzw. Y von der Mindest-Empfindlichkeit T subtrahiert, da eine höhere Empfindlichkeit S in der Regel einem kleineren Zahlenwert der Empfindlichkeit S entspricht. In der Fig. 5 bedeutet die Angabe SENSE verbunden mit der Angabe einer bestimmten Empfindlichkeit S übrigens immer, dass die Empfindlichkeit S der ersten Detektoreinrichtung (27) auf den jeweiligen Wert eingestellt ist.

Im Zuge der selbsttätigen Einstellung der Empfindlichkeit S der ersten Detektoreinrichtung (27), die zu jedem ersten Zeitpunkt erfolgt, wird die zweite Detektoreinrichtung (43) aktiviert. Dann wird für eine definierte Anzahl von mittels der zweiten Detektoreinrichtung (43) detektierten natürlichen Herzschlägen, in Fig. 5 sind es beispielsweise vier natürliche Herzschläge, überprüft, ob diese auch mittels der ersten Detektoreinrichtung (27) detektiert werden, die auf die Empfindlichkeit S = T-X eingestellt ist, und zwar unabhängig davon, ob zuvor im "NORMAL MODE" auch das zusätzliche Sicherheits-Marginal Y vorhanden war. Die Detektion eines natürlichen Herzschlages mittels der ersten Detektoreinrichtung (27) ist in Fig. 5 durch die Angabe DET veranschaulicht. Bleibt die Detektion eines mittels der zweiten Detektoreinrichtung (43) detektierten Herzschlages durch die erste Detektoreinrichtung (27) aus, ist dies durch die Angabe NO DET verdeutlicht.

Werden alle natürlichen Herzschläge der definierten Anzahl von mittels der zweiten Detektoreinrichtung (43) detektierten natürlichen Herzschlägen auch mittels der ersten Detektoreinrichtung (27) detektiert, wird die Empfindlichkeit S der ersten Detektoreinrichtung (27) auf die zuletzt gefundene Mindest-Empfindlichkeit T eingestellt. Detektiert dann die erste Detektoreinrichtung (27) den nächsten mittels der zweiten Detektoreinrichtung (43) detektierten natürlichen Herzschlag, wird die Empfindlichkeit S der ersten Detektoreinrichtung (27) nochmals um einen definierten Schritt E auf S = T+E verringert. Der Schritt E kann z.B ein bestimmter Bruchteil oder ein Vielfaches der maximal möglichen Empfindlichkeit Sₘₐₓ der ersten Detektoreinrichtung (27) sein. Detektiert die erste Detektoreinrichtung (27) mit der Empfindlichkeit S = T+E den nächsten mittels der zweiten Detektoreinrichtung (43) detektierten natürlichen Herzschlag ebenfalls, wird als neue Mindest-Empfindlichkeit T der ersten Detektoreinrichtung (27) T = T+E und als neue Empfindlichkeit S der ersten Detektoreinrichtung (27) S = T-X gesetzt, was durch die Angaben SET T = T+E und SET S = T-X verdeutlicht ist. Im übrigen bedeutet in der Fig. 5 die Angabe SET in Verbindung mit der Angabe eines Parameters immer, dass eine neue Festsetzung des entsprechenden Parameters auf den angegebenen Wert erfolgt. Auf die erwähnte neue Empfindlichkeit S, die wohlgemerkt um den Schritt E geringer ist, als die zuvor vorhandene Empfindlichkeit, wird die erste Detektoreinrichtung (27) dann im "NORMAL MODE" eingestellt.

Es wäre zwar möglich, dass sich die Verhältnisse zwischen zwei aufeinanderfolgenden ersten Zeitpunkten so stark ändern, dass eine Verringerung der Mindest-Empfindlichkeit T um mehr als einen Schritt E möglich wäre. Dennoch wird im Interesse der Sicherheit des Patienten eine Verringerung nur um einen Schritt E vorgenommen. Sollten sich die Verhältnisse tatsächlich auf einem Niveau stabilisieren, das eine weitere Verringerung der Empfindlichkeit S der zweiten Detektoreinrichtung (27) gestattet, so erfolgt diese im Zuge der nächsten selbsttätigen Einstellung(en) der Empfindlichkeit S.

Detektiert die erste Detektoreinrichtung (27) mit der Empfindlichkeit S = T+E den nächsten mittels der zweiten Detektoreinrichtung (43) detektierten natürlichen Herzschlag nicht, bedeutet dies, dass sich die Verhältnisse seit dem letzten ersten Zeitpunkt nicht verändert haben. Daher werden die Mindest-Empfindlichkeit T und die Empfindlichkeit S wieder auf die zuvor vorhandenen Werte eingestellt, bevor die Rückkehr in den "NORMAL MODE" erfolgt.

Detektiert die erste Detektoreinrichtung (27) bereits mit der zuletzt vorhandenen Mindest-Empfindlichkeit T einen mittels der zweiten Detektoreinrichtung (43) detektierten natürlichen Herzschlag nicht, bedeutet dies, dass eine Erhöhung der Mindest-Empfindlichkeit T erfolgen muss. Um die ordnungsgemässe Funktion des Herzschrittmachers im Interesse des Patienten möglichst weitgehend aufrechterhalten zu können, wird jedoch zunächst bis zur Detektion des nächsten natürlichen Herzschlages die Empfindlichkeit S auf ihren maximalen Wert Sₘₐₓ eingestellt, der jedoch so gewählt ist, dass Störungen und Muskelzuckungen nicht zu Fehldetektionen führen können. Wird der nächste mittels der zweiten Detektoreinrichtung (43) detektierte natürliche Herzschlag auch mit der auf maximale Empfindlichkeit Sₘₐₓ eingestellten ersten Detektoreinrichtung (27) detektiert, wird die erste Detektoreinrichtung (27)auf eine Empfindlichkeit S eingestellt, die der zuletzt vorhandenen, um den Schritt E erhöhten Mindest-Empfindlichkeit T entspricht. Detektiert die erste Detektoreinrichtung (27) mit dieser Empfindlichkeit S den nächsten mittels der zweiten Detektoreinrichtung (43) detektierten natürlichen Herzschlag, wird die neue Mindest-Empfindlichkeit T entsprechend festgesetzt. Als neue Empfindlichkeit S wird jedoch anders als zuvor nicht nur die um das Sicherheits-Marginal X erhöhte Mindest-Empfindlichkeit T eingestellt. Vielmehr erfolgt eine weitere Erhöhung um das bereits erwähnte zusätzliche Sicherheits-Marginal Y. Bis zum nächsten ersten Zeitpunkt ist also im "NORMAL MODE" die Empfindlichkeit der ersten Detektoreinrichtung (27) auf S = T-X-Y eingestellt. Dies geschieht, um für den Fall, dass eine weitere Erhöhung der Mindest-Empfindlichkeit T erforderlich werden sollte, bereits entsprechende Vorkehrungen im Interesse der Sicherheit des Patienten zu treffen. Für den Fall, dass bis zum nächsten ersten Zeitpunkt keine weitere Erhöhung der Mindest-Empfindlichkeit T erforderlich wird, entfällt, wie sich aus den vorstehenden Ausführungen ergibt, das zusätzliche Sicherheits-Marginal Y wieder.

Reicht die um einen Schritt E erhöhte Mindest-Empfindlichkeit T der ersten Detektoreinrichtung (27) nicht aus, um einen mittels der zweiten Detektoreinrichtung (43) detektierien natürlichen Herzschlag ebenfalls zu detektieren, wird die Mindest-Empfindlichkeit T um einen weiteren Schritt E erhöht. Zunächst wird aber die Empfindlichkeit der ersten Detektoreinrichtung (27) auf ihren maximalen Wert Sₘₐₓ eingestellt, um die Detektion des nächsten natürlichen Herzschlages zu ermöglichen. Erst wenn diese Detektion erfolgt ist, wird für den darauffolgenden mittels der zweiten Detektoreinrichtung (43) detektierten natürlichen Herzschlag überprüft, ob eine Detektion auch mittels der ersten Detektoreinrichtung (27) mit der um einen weiteren Schritt E erhöhten Empfindlichkeit S erfolgt. Dies wiederholt sich solange, bis eine neue Mindest-Empfindlichkeit T gefunden ist, mit der die erste Detektoreinrichtung (27) einen mittels der zweiten Detektoreinrichtung (43) detektierten natürlichen Herzschlag ebenfalls detektiert. Daraufhin wird die neue Mindest-Empfindlichkeit T und die neue Empfindlichkeit S wie unmittelbar vorstehend beschrieben gesetzt und mit diesen Werten in den "NORMAL MODE" übergegangen, wobei dann das Sicherheits-Marginal X und das zusätzliche Sicherheits-Marginal Y vorhanden sind.

Wird ein mittels der zweiten Detektoreinrichtung (43) detektierter natürlicher Herzschlag mit der ersten Detektoreinrichtung (27) selbst dann nicht detektiert, wenn diese auf ihre maximale Empfindlichkeit Sₘₐₓ eingestellt ist, wird für den weiteren Betrieb des Herzschrittmachers die Empfindlichkeit S der ersten Detektoreinrichtung (27) auf ihren maximalen Wert Sₘₐₓ eingestellt. Ausserdem werden Massnahmen getroffen, die sicherstellen, dass bei der nächsten Kommunikation des Programmers (37) mit dem Herzschrittmacher (1) der behandelnde Arzt darauf hingewiesen wird, dass die Empfindlichkeit der ersten Detektoreinrichtung (27) auf ihren maximalen Wert Sₘₐₓ eingestellt wurde. Anstelle der Einstellung der Empfindlichkeit der ersten Detektoreinrichtung auf ihren maximalen Wert Sₘₐₓ kann auch vorgesehen sein, dass die erste Detektoreinrichtung (27) deaktiviert wird und die zweite Detektoreinrichtung (43) an ihre Stelle tritt.

Detektiert die erste Detektoreinrichtung (27), während ihre Empfindlichkeit S auf den Wert S = T-X eingestellt ist, zu Anfang des beschriebenen Betriebsverfahrens einen der vier mittels der zweiten Detektoreinrichtung (43) detektierten natürlichen Herzschläge nicht, bedeutet dies, dass die Mindest-Empfindlichkeit T der ersten Detektoreinrichtung (27) um mehr als das Sicherheits-Marginal X erhöht werden muss, um überhaupt eine Detektion eines mittels der zweiten Detektoreinrichtung (43) detektierten natürlichen Herzschlages mit der ersten Detektoreinrichtung (27) zu ermöglichen. In diesem Falle wird daher zunächst eine neue Mindest-Empfindlichkeit T festgesetzt, die der zuvor eingestellten Empfindlichkeit S entspricht. Im Anschluss daran wird zur Ermittlung der Mindest-Empfindlichkeit T in der zuvor beschriebenen Weise verfahren, worauf nach Einstellung einer entsprechenden Empfindlichkeit S die Rückkehr in den "NORMAL MODE" erfolgt.

Zwischen zwei aufeinanderfolgenden ersten Zeitpunkten wird im Falle der selbsttätigen Einstellung der Empfindlichkeit S der ersten Detektoreinrichtung (27) ähnlich wie im Falle der selbsttätigen Einstellung des Energiegehaltes A der Stimulationsimpulse zu wenigstens einem zweiten Zeitpunkt, sind mehrere zweite Zeitpunkte vorgesehen, liegen zwischen diesen Zeitintervalle in der Grössenordnung von Minuten, geprüft, ob die im "NORMAL MODE" vorhandene Einstellung der Empfindlichkeit S im wesentlichen noch korrekt ist. Zu diesem Zweck wird der "NORMAL MODE" verlassen und die zweite Detektoreinrichtung (43) aktiviert. Es wird dann für eine definierte Anzahl von mittels der zweiten Detektoreinrichtung (43) detektierten natürlichen Herzschlägen, im Falle der FIG 5 sind dies vier natürliche Herzschläge, geprüft ob diese Herzschläge auch mittels der ersten Detektoreinrichtung (27) detektiert werden. Ist dies für alle natürlichen Herzschläge der definierten Anzahl der Fall, erfolgt die Rückkehr in den "NORMAL MODE". Bleibt für einen der natürlichen Herzschläge die Detektion mittels der ersten Detektoreinrichtung (27) aus, wird zunächst die Mindest-Empfindlichkeit T neu festgesetzt, und zwar auf einen Wert, der der zuvor in "NORMAL MODE" vorhandenen Empfindlichkeit S entspricht. Im Anschluss daran erfolgt die Ermittlung einer neuen Mindest-Empfindlichkeit T in der zuvor schon beschriebenen Weise, und nach der Festsetzung und Einstellung einer neuen Empfindlichkeit S für die erste Detektoreinrichtung (27) die Rückkehr in den "NORMAL MODE".

Die ersten und zweiten Zeitpunkte werden zweckmässigerweise ermittelt, indem der Mikroprozessor (5) eine entsprechend Anzahl von mittels des Quarzes (14) erzeugten Taktimpulsen abzählt.

Wesentliche Funktionen des Herzschrittmachers gemäss dem beschriebenen Ausführungsbeispiel sind durch einen geeignet programmierten Mikroprozessor (5) gesteuert. Die entsprechenden Funktionen können jedoch ohne weiteres auch durch eine herkömmlich aufgebaute Steuerlogik realisiert werden.

Bei der beschriebenen Ausführungsform der zweiten Detektoreinrichtung (43) handelt es sich um eine bevorzugte Ausführungsform dieser Detektoreinrichtung. Es können jedoch auch andere geeignete Detektoreinrichtungen Verwendung finden, die ebenfalls natürliche Herzschläge in einem dem zeitlichen Verlauf der elektrischen Impedanz des Herzens entsprechenden Signal detektieren.

Obwohl die Erfindung ausschliesslich anhand eines Herzschrittmachers erläutert ist, kann sie auch bei anderen medizinischen Geräten Verwendung finden, die eine Detektoreinrichtung mit einstellbarer Empfindlichkeit aufweisen.

## Patentansprüche

1. In den Körper eines Lebewesens implantierbares medizinisches Gerät mit einer ersten Detektoreinrichtung (27) zur Detektion von natürlichen Herzschlägen in einem der elektrischen Aktivität des Herzens entsprechenden Signal (ES), mit Stellmitteln (5, 31) zur Einstellung der Empfindlichkeit (S) der ersten Detektoreinrichtung (27) and mit einer zweiten Detektoreinrichtung (43) zur Detektion von natürlichen Herzschlägen in einem dem zeitlichen Verlauf der elektrischen Impedanz des Herzens entsprechenden Signal (IS), deren Empfindlichkeit mindestens der jeweils eingestellten Empfindlichkeit (S) der ersten Detektoreinrichtung (27) entspricht, wobei die Stellmittel (5, 31) die Empfindlichkeit (S) der ersten Detektoreinrichtung (27) selbsttätig derart einstellen, dass die erste Detektoreinrichtung (27) die mittels der zweiten Detektoreinrichtung (43) detektierten Herzschläge detektiert, und wobei die zweite Detektoreinrichtung (43) nur als Referenz für die erste Detektoreinrichtung (27) dient.

2. Gerät nach Anspruch 1, **dadurch gekennzeichnet, dass** die Stellmittel (5, 31) die Empfindlichkeit (S) der ersten Detektoreinrichtung (27) auf einen Wert einstellen, der um ein Sicherheits-Marginal (X, Y) der Empfindlichkeit (S) höher als ein Mindestwert (T) der Empfindlichkeit (S) ist, bei dem die erste Detektoreinrichtung (27) jeden mittels der zweiten Detektoreinrichtung (43) detektierten natürlichen Herzschlag detektiert.

3. Gerät nach Anspruch 2, **dadurch gekennzeichnet, dass** die Stellmittel (5, 31) zur Ermittlung des Mindestwertes (T) die Empfindlichkeit (S) der ersten Detektoreinrichtung (27) ausgehend von einem Wert, bei dem die erste Detektoreinrichtung (27) wenigstens einzelne mittels der zweiten Detektoreinrichtung (43) detektierte natürliche Herzschläge nicht detektiert, allmählich gerade soweit erhöhen, bis die erste Detektoreinrichtung (27) jeden mittels der zweiten Detektoreinrichtung (43) detektierten natürlichen Herzschlag detektiert, und dass die Stellmittel (5, 31) zur Ermittlung desjenigen Wertes der Empfindlichkeit (S) der ersten Detektoreinrichtung (27), bei dem diese wenigstens einzelne mittels der zweiten Detektoreinrichtung (43) detektierte natürlichen Herzschläge nicht detektiert, die Empfindlichkeit (S) der ersten Detektoreinrichtung (27) ausgehend von einem Wert, bei dem die erste Detektoreinrichtung (27) jeden mittels der zweiten Detektoreinrichtung (43) detektierten natürlichen Herzschlag detektiert, allmählich verringern.

4. Gerät nach einem der Ansprüche 1 bis 4, **dadurch gekennzeichnet, dass** das Gerät als Herzschrittmacher ausgebildet ist.

5. Gerät nach Anspruch 4, **dadurch gekennzeichnet, dass** die zweite Detektoreinrichtung (43) eine modulierbare Stromquelle (52), eine Demodulatorschaltung (56), eine mit der Stromquelle (52) und der Demodulatorschaltung (56) verbundene Oszillatorschaltung (53), die ein periodisches Oszillatorsignal (P) erzeugt, und eine Detektorschaltung (57, 58) aufweist, wobei das Oszillatorsignal (P) die Stromquelle (52) derart moduliert, dass diese einen Wechselstrom (Q) konstante Amplitude abgibt, der mit dem Oszillatorsignal (P) synchronisiert ist, wobei der Wechselspannungsanteil (R) der über der Stromquelle (52) abfallenden Spannung der Demodulatorschaltung (56) zugeführt ist, die mittels des Oszillatorsignals (P) mit der Stromquelle (52) synchronisiert ist und den Wechselspannungsanteil (R) demoduliert, und wobei das demodulierte Signal (U) der Detektorschaltung (57, 58) zugeführt ist.

6. Gerät nach Anspruch 5, **dadurch gekennzeichnet, dass** die Stromquelle (52) der zweiten Detektoreinrichtung (43) zwei Anschlüsse aufweist, an die eine bipolare Elektrode (3) anschliessbar ist.

7. Verfahren zum Betrieb eines Gerätes nach einem der Ansprüche 1 bis 6, **dadurch gekennzeichnet, dass** zu aufeinanderfolgenden ersten Zeitpunkten die Empfindlichkeit (S) der ersten Detektoreinrichtung (27) selbsttätig eingestellt wird.

8. Verfahren nach Anspruch 7, **dadurch gekennzeichnet, dass** zu wenigstens einem zwischen zwei aufeinanderfolgenden ersten Zeitpunkten liegenden zweiten Zeitpunkt selbsttätig geprüft wird, ob ein mittels der zweiten Detektoreinrichtung (43) detektierter Herzschlag mittels der ersten Detektoreinrichtung (27) mit der zuletzt eingestellten Empfindlichkeit ebenfalls detektiert wird, und dass, falls dies nicht der Fall ist, die Empfindlichkeit (S) der ersten Detektoreinrichtung (27) selbsttätig eingestellt wird.

9. Verfahren nach Anspruch 7 oder 8, dass bei der selbsttätigen Einstellung der Empfindlichkeit (S) der ersten Detektoreinrichtung (27) der Mindestwert (T) der Empfindlichkeit (S) ermittelt wird.

10. Verfahren nach Anspruch 9, dass als Empfindlichkeit (S) der ersten Detektoreinrichtung (27) eine Empfindlichkeit eingestellt wird, die um ein Sicherheits-Marginal (X) höher als der Mindestwert (T) der Empfindlichkeit (S) ist.

11. Verfahren nach Anspruch 10, **dadurch gekennzeichnet, dass**, falls ein Mindestwert (T) der Empfindlichkeit (S) der ersten Detektoreinrichtung (27) ermittelt wird, der höher als der zuletzt ermittelter Mindestwert (T) ist, eine Empfindlichkeit eingestellt wird, die um das Sicherheits-Marginal (X) and ein zusätzliches Sicherheits-Marginal (Y) höher als der der Mindestwert (T) der Empfindlichkeit (S) ist.

12. Verfahren nach einem der Ansprüche 7 bis 11, **dadurch gekennzeichnet, dass** falls ein Mindestwert (T) der Empfindlichkeit (S) der ersten Detektoreinrichtung (27) ermittelt wird, der höchstens gleich dem zuletzt ermittelten Mindestwert (T) ist, bei der anschliessenden Einstellung der Empfindlichkeit (S) ein zuvor gegebenenfalls vorhandenes zusätzliches Sicherheits-Marginal (Y) der Empfindlichkeit (S) entfällt.

13. Verfahren nach einem der Ansprüche 9 bis 11, **dadurch gekennzeichnet, dass** zur Ermittlung des Mindestwertes (T) der Empfindlichkeit (S) der ersten Detektoreinrichtung (27) geprüft wird, ob ein mittels der zweiten Detektor (43) detektierter natürlicher Herzschlag auch mittels der ersten Detektoreinrichtung (27) mit auf den zuletzt ermittelten Mindestwert (T) eingestellter Empfindlichkeit (S) detektiert wird, und dass, wenn dies nicht der Fall ist, die Empfindlichkeit (S) solange jeweils um einen definierten Schritt (E) erhöht wird, bis ein mittels der zweiten Detektoreinrichtung (43) detektierter natürlicher Herzschlag auch mittels der ersten Detektoreinrichtung (27) mit der auf den erhöhten Mindestwert (T) eingestellten Empfindlichkeit (S) detektiert wind, worauf der Einstellung der Empfindlichkeit (S) der ersten Detektoreinrichtung (27) der erhöhte Mindestwert (T) zugrunde gelegt wird.

14. Verfahren nach einem der Ansprüche 9 bis 12, **dadurch gekennzeichnet, dass** zur Ermittlung des Mindestwertes (T) der Empfindlichkeit (S) der ersten Detektoreinrichtung (27) geprüft wird, ob ein mittels der zweiten Detektoreinrichtung (43) detektierter natürlicher Herzschlag auch mittels der ersten Detektoreinrichtung (27) mit auf den zuletzt ermittelten Mindestwert (T) eingestellter Empfindlichkeit (S) detektiert wird, und dass, wenn dies der Fall ist, die Empfindlichkeit (S) um einen definierten Schritt (E) verringert wird, wobei für den Fall, dass ein mittels der zweiten Detektoreinrichtung (43) detektierter natürlicher Herzschlag auch mittels der ersten Detektoreinrichtung (27) mit der auf den verringerten Mindestwert eingestellten Empfindlichkeit (S) detektiert wird, der verringerte Mindestwert der Einstellung der Empfindlichkeit (S) der ersten Detektoreinrichtung (27) als Mindestwert (T) zugrunde gelegt wind, während anderenfalls der zuletzt ermittelte Mindestwert (T) beibehalten und der Einstellung der Empfindlichkeit (S) zugrunde gelegt wird.

15. Verfahren nach einem der Ansprüche 9 bis 13, **dadurch gekennzeichnet, dass** bei der selbsttätigen Einstellung der Empfindlichkeit (S) der ersten Detektoreinrichtung (27) vor der Ermittlung des Mindestwertes (T) der Empfindlichkeit (S) geprüft wird, ob ein mittels der zweiten Detektoreinrichtung detektierter natürlicher Herzschlag auch mittels der ersten Detektoreinrichtung (27) mit der zuletzt eingestellten Empfindlichkeit, verringert um das gegebenenfalls vorhandene zusätzliche Sicherheits-Marginal (Y), ebenfalls detektiert wird, und dass, wenn dies nicht der Fall ist, bei der selbsttätigen Einstellung der Empfindlichkeit (S) der ersten Detektoreinrichtung (27) als zuletzt ermittelter Mindestwert (T) der Empfindlichkeit, verringert um das gegebenenfalls vorhandene zusätzliche Sicherheits-Marginal (Y), zugrunde gelegt wird.

16. Verfahren nach einem der Ansprüche 9 bis 13, **dadurch gekennzeichnet, dass** falls zu einem zweiten Zeitpunkt der mittels der zweiten Detektoreinrichtung (43) detektierte natürliche Herzschlag nicht ebenfalls mittels der ersten Detektoreinrichtung (27) detektiert wind, bei der selbsttätigen Einstellung der Empfindlichkeit (S) der ersten Detektoreinrichtung (27) als zuletzt ermittelter Mindestwert (T) der Empfindlichkeit (S) der zuletzt eingestellte Wert der Empfindlichkeit zugrunde gelegt wird.

17. Verfahren nach einem der Ansprüche 9 bis 13 oder 16, **dadurch gekennzeichnet, dass** für den Fall, dass ein mittels der zweiten Detektoreinrichtung (43) detektierter natürlicher Herzschlag nicht auch mit der ersten Detektoreinrichtung (27) detektiert wind, die Empfindlichkeit (S) der ersten Detektoreinrichtung (27) für die Detektion des nächsten natürlichen Herzschlages auf ihren Maximalwert (Sₘₐₓ) eingestellt wird.

## Claims

1. Medical device which is implantable in the body of a living organism and which has a first detector device (27) for the detection of natural heartbeats in a signal (ES) corresponding to the electrical activity of the heart, having regulating means (5, 31) for the setting of the sensitivity (S) of the first detector device (27) and having a second detector device (43) for the detection of natural heartbeats in a signal (IS) corresponding to the temporal progression of the electrical impedance of the heart, the sensitivity of which at least corresponds to the respectively set sensitivity (S) of the first detector device (27), the regulating means (5, 31) automatically setting the sensitivity (S) of the first detector device (27) in such a manner that the first detector device (27) detects the heartbeats detected by means of the second detector device (43), and wherein the second detector device (43) serves only as a reference for the first detector device (27).

2. Device according to Claim 1, **characterized in that** the regulating means (5, 31) set the sensitivity (S) of the first detector device (27) to a value which is greater, by a safety margin (X, Y) of the sensitivity (S), than a minimum value (T) of the sensitivity (S), at which the first detector device (27) detects each detected natural heartbeat detected by means of the second detector device (43).

3. Device according to Claim 2, **characterized in that** to determine the minimum value (T) the regulating means (5, 31) gradually increase the sensitivity (S) of the first detector device (27), starting from a value at which the first detector device (27) does not detect at least individual natural heartbeats detected by means of the second detector device (43) just until such time as the first detector device (27) detects each natural heartbeat detected by means of the second detector device (43), and **in that** to determine that value of the sensitivity (S) of the first detector device (27) at which the latter does not detect at least individual natural heartbeats detected by means of the second detector device (43), the regulating means (5, 31) gradually reduce the sensitivity (S) of the first detector device (27), starting from a value at which the first detector device (27) detects each natural heartbeat detected by means of the second detector device (43).

4. Device according to one of Claims I to 3, **characterized in that** the device is designed as a cardiac pacemaker.

5. Device according to Claim 4, **characterized in that** the second detector device (43) exhibits a modulatable current source (52), a demodulator circuit (56), an oscillator circuit (53) which is connected to the current source (52) and the demodulator circuit (56) and which generates a periodic oscillator signal (P), and a detector circuit (57, 58), the oscillator signal (P) modulating the current source (52) in such a manner that the latter gives off an alternating current (Q) of constant amplitude, which alternating current is synchronized with the oscillator signal (P), the alternating voltage component (R) of the voltage dropping over the current source (52) being fed to the demodulator circuit (56), which is synchronized with the current source (52) by means of the oscillator signal (P) and demodulates the alternating voltage component (R), and the demodulated signal (U) being fed to the detector circuit (57, 58).

6. Device according to Claim 5, **characterized in that** the current source (52) of the second detector device (43) exhibits two connections to which a bipolar electrode (3) is connectable.

7. Process for the operation of a device according to one of Claims 1 to 6, **characterized in that** at successive first instants the sensitivity (S) of the first detector device (27) is automatically set.

8. Process according to Claim 7, **characterized in that** at at least one second instant situated between two successive first instants a check is automatically made as to whether a natural heartbeat detected by means of the second detector device (43) is likewise detected by means of the first detector device (27) with the last set sensitivity, and **in that**, if this is not the case, the sensitivity (S) of the first detector device (27) is automatically set.

9. Process according to Claim 7 or 8, **characterized in that** in the automatic setting of the sensitivity (S) of the first detector device (27) the minimum value (T) of the sensitivity (S) is determined.

10. Process according to Claim 9, **characterized in that** as sensitivity (S) of the first detector device (27) there is set a sensitivity which is greater, by a safety margin (X), than the minimum value (T) of the sensitivity (S).

11. Process according to Claim 10, **characterized in that**, in the event that a minimum value (T) of the sensitivity (S) of the first detector device (27) is determined which is greater than the last determined minimum value (T), a sensitivity is set, which is greater, by the safety margin (X) and an additional safety margin (Y), than the minimum value (T) of the sensitivity (S).

12. Process according to one of Claims 7 to 11, **characterized in that**, in the event that a minimum value (T) of the sensitivity (S) of the first detector device (27) is determined which is at most equal to the last determined minimum value (T), in the course of the subsequent setting of the sensitivity (S) a possibly previously present additional safety margin (Y) of the sensitivity (S) is dispensed with.

13. Process according to one of Claims 9 to 11, **characterized in that** to determine the minimum value (T) of the sensitivity (S) of the first detector device (27) a check is made as to whether a natural heartbeat detected by means of the second detector device (43) is also detected by means of the first detector device (27) with a sensitivity (S) set to the last determined minimum value (T), and **in that**, if this is not the case, the sensitivity (S) is increased in each instance by a defined step (E) until such time as a natural heartbeat detected by means of the second detector device (43) is also detected by means of the first detector device (27) with the sensitivity (S) set to the increased minimum value (T), whereupon the increased minimum value (T) is adopted as a basis for the setting of the sensitivity (S) of the first detector device (27).

14. Process according to one of Claims 9 to 12, **characterized in that** to determine the minimum value (T) of the sensitivity (S) of the first detector device (27) a check is made as to whether a natural heartbeat detected by means of the second detector device (43) is also detected by means of the first detector device (27) with a sensitivity (S) set to the last determined minimum value (T), and **in that**, if this is the case, the sensitivity (S) is reduced by a defined step (E), in which process, in the event that a natural heartbeat detected by means of the second detector device (43) is also detected by means of the first detector device (27) with the sensitivity (S) set to the reduced minimum value, the reduced minimum value is adopted as a basis as minimum value (T) for the setting of the sensitivity (S) of the first detector device (27), while otherwise the last determined minimum value (T) is retained and is adopted as a basis for the setting of the sensitivity (S).

15. Process according to one of Claims 9 to 13, **characterized in that** in the automatic setting of the sensitivity (S) of the first detector device (27) before the determination of the minimum value (T) of the sensitivity (S) a check is made as to whether a natural heartbeat detected by means of the second detector device (43) is also likewise detected by means of the first detector device (27) with the last set sensitivity, reduced by the possibly present additional safety margin (Y), and **in that**, if this is not the case, in the automatic setting of the sensitivity (S) of the first detector device (27) the last set sensitivity, reduced by the possibly present additional safety margin (Y), is adopted as a basis as the last determined minimum value (T) of the sensitivity (S).

16. Process according to one of Claims 9 to 13, **characterized in that**, if at a second instant the natural heartbeat detected by means of the second detector device (43) is not likewise detected by means of the first detector device (27), in the automatic setting of the sensitivity (S) of the first detector device (27) the last set value of the sensitivity is adopted as a basis as the last determined minimum value (T) of the sensitivity (S).

17. Process according to one of Claims 9 to 13 or 16, **characterized in that**, in the event that a natural heartbeat detected by means of the second detector device (43) is not also detected by the first detector device (27), the sensitivity (S) of the first detector device (27) is set to its maximum value (Sₘₐₓ) for the detection of the next natural heartbeat.

## Revendications

1. Appareil médical implantable dans le corps d'un être vivant, comportant un premier dispositif (27) de détection pour la détection de battements cardiaques naturels dans un signal (ES) correspondant à l'activité électrique du coeur, comportant des moyens (5, 31) de réglage pour régler la sensibilité (S) du premier dispositif de détection (27) et comportant un deuxième dispositif (43) de détection pour détecter des battements cardiaques naturels dans un signal (IS) correspondant à la courbe temporelle de l'impédance électrique du coeur, dont la sensibilité correspond au moins à la sensibilité (S) réglée du premier dispositif (27) de détection, les moyens de réglage (5, 31) réglant automatiquement la sensibilité (S) du premier dispositif (27) de détection de telle sorte que le premier dispositif (27) de détection détecte des battements cardiaques détectés au moyen du deuxième dispositif (43) de détection, et dans lequel le deuxième dispositif (43) de détection sert seulement de référence pour le premier dispositif (27) de détection.

2. Appareil suivant la revendication 1, **caractérisé en ce que** les moyens de réglage (5, 31) règlent la sensibilité (S) du premier dispositif (27) de détection à une valeur qui est supérieure, dune marge (X, Y) de sécurité de la sensibilité (S), à une valeur minimale (T) de la sensibilité (S), le premier dispositif (27) de détection détectant chaque battement cardiaque naturel détecté au moyen du deuxième dispositif (43) de détection.

3. Appareil suivant la revendication 2, **caractérisé en ce que** les moyens (5, 31) de réglage destinés à déterminer la valeur (T) minimale augmentent progressivement la sensibilité (S) du premier dispositif (27) de détection à partir d'une valeur, pour laquelle le premier dispositif (27) de détection ne détecte pas de battement cardiaque naturel détecté uniquement au moyen du deuxième dispositif (43) de détection, précisément jusqu'à ce que le premier dispositif (27) de détection détecte chaque battement cardiaque naturel détecté au moyen du deuxième dispositif (43) de détection, et **en ce que** les moyens de réglage (5, 31) destinés à déterminer chaque valeur de la sensibilité (S) du premier dispositif (27) de détection, pour laquelle ce dispositif de détection ne détecte pas au moins des battements cardiaques naturels détectés par le deuxième dispositif (43) de détection, réduisent progressivement la sensibilité (S) du premier dispositif (27) de détection à partir d'une valeur, pour laquelle le premier dispositif de détection (27) détecte chaque battement cardiaque naturel détecté au moyen du deuxième dispositif (43) de détection.

4. Appareil suivant l'une des revendications 1 à 4, **caractérisé en ce que** l'appareil est réalisé sous la forme d'un stimulateur cardiaque.

5. Appareil suivant la revendication 4, **caractérisé en ce que** le deuxième dispositif de détection (43) comporte une source (52) de courant modulable, un circuit démodulateur (56), un circuit (53) oscillateur, qui est raccordé à la source de courant (52) et au circuit démodulateur (56), et qui produit un signal périodique (P) d'oscillation, et un circuit de détection (57, 58), le signal (P) de l'oscillateur modulant la source de courant (52) de telle sorte que cette derrière délivre un courant (Q) alternatif d'amplitude constante, qui est synchronisé avec le signal (P) d'oscillateur, la composante (R) de tension alternative de la tension qui chute dans la source de courant (52) étant envoyée au circuit modulateur (56) qui est synchronisée sur la source de courant (52) au moyen du signal (P) d'oscillateur, et démodulant la composante de tension alternative (R), le signal démodulé (U) étant envoyé au circuit de détection (57, 58).

6. Appareil suivant la revendication 5, **caractérisé par le fait que** la source de courant (52) du deuxième dispositif (43) de détection comporte deux bornes, à laquelle peut être raccordée une électrode (3) bipolaire.

7. Procédé pour faire fonctionner un appareil suivant l'une des revendications 1 à 6, **caractérisé en ce que** la sensibilité (S) du premier dispositif de détection (27) est réglée automatiquement à des premiers instants qui se suivent.

8. Procédé suivant la revendication 7, **caractérisé en ce qu'**au moins à un second instant dans le temps situé entre deux premiers instants successifs, il est vérifié automatiquement le fait de savoir si un battement cardiaque détecté au moyen du deuxième dispositif (43) de détection est détecté également au moyen du premier dispositif (27) de détection avec la sensibilité réglée en dernier lieu, et **en ce que**, si ce n'est pas le cas, la sensibilité (S) du premier dispositif (27) de détection est réglée automatiquement.

9. Procédé suivant la revendication 7 ou 8, **caractérisé en ce que** lors du réglage automatique de la sensibilité (S), du premier dispositif (27) de détection, il est déterminé la valeur (T) minimum de la sensibilité (S).

10. Procédé suivant la revendication 9, **caractérisé en ce que** l'on règle comme sensibilité (S) du premier dispositif (27) de détection une sensibilité qui est supérieure, d'une marge de sécurité (X) à la valeur minimale (T) de la sensibilité (S).

11. Procédé suivant la revendication 10, **caractérisé en ce que** dans le cas où il est déterminée une valeur (T) minimale de la sensibilité (S) du premier dispositif de détection (27), qui est supérieure à la valeur (T) minimum déterminée en dernier lieu, il est réglée une sensibilité, qui est, d'une marge (X) de sécurité et d'une marge (Y) de sécurité supplémentaire, supérieure à la valeur (T) minimum de la sensibilité (S).

12. Procédé suivant l'une des revendications 7 à 11, **caractérisé par le fait que** dans le cas où il est déterminé une valeur (T) minimum de la sensibilité (S) du premier dispositif (27) de détection, qui est au maximum égale à la valeur (T) minimale déterminée en dernier lieu, une marge de sécurité (Y) supplémentaire éventuellement présente auparavant de la sensibilité (S) est supprimée lors du réglage ultérieur de la sensibilité (S).

13. Procédé suivant Tune des revendications 9 à 11, **caractérisé en ce que** pour la détermination de la valeur minimale (T) de la sensibilité (S) du premier dispositif (27) de détection, il est vérifié pour savoir si un battement cardiaque naturel détecté par le deuxième détecteur (43) est également détecté au moyen du premier dispositif de détection (27) avec une sensibilité (S) réglée à la valeur (T) minimale déterminée en dernier lieu, et **en ce que**, si ce n'est pas le cas, la sensibilité (S) est accrue respectivement d'un pas (E) défini jusqu'à ce qu'un battement cardiaque naturel détecté au moyen du deuxième dispositif (43) de détection soit également détecté au moyen du premier dispositif (27) de détection avec la sensibilité (S) réglée à la valeur (T) minimale accrue, à la suite de quoi le réglage de la sensibilité (S) du premier dispositif (27) de détection est effectué sur la base de la valeur minimale (T) accrue.

14. Procédé suivant l'une des revendications 9 à 12, **caractérisé par le fait que** pour la détermination de la valeur minimale (T) de la sensibilité (S) du premier dispositif de détection (27), il est vérifié le point de savoir si un battement cardiaque naturel détecté au moyen du deuxième dispositif de détection (43) est également détecté par le premier dispositif (27) de détection avec une sensibilité (S) réglée à la valeur minimale (T) déterminée en dernier lieu et si c'est le cas, la sensibilité (S) est réduite d'un pas défini, auquel cas, lorsqu'un battement cardiaque naturel est détecté au moyen du deuxième dispositif (43) de détection par le deuxième dispositif (43) de détection est également détecté par le première dispositif (27) de détection avec la sensibilité (S) réglée à la valeur minimale réduite, on prend comme valeur minimale (T) la valeur minimale réduite pour le réglage de la sensibilité (S) du premier dispositif de détection tandis que sinon, on conserve la valeur minimale (T) déterminée en dernier lieu et on l'utilise pour le réglage de la sensibilité (S).

15. Procédé suivant l'une des revendications 9 à 13, **caractérisé en ce que** lors du réglage automatique de la sensibilité (S) du premier dispositif (27) de détection, avant la détermination de la valeur minimale (T) de la sensibilité (S), il est vérifié pour savoir si un battement cardiaque naturel détecté au moyen du deuxième dispositif de détection est également détecté au moyen du premier dispositif de détection (27) avec la sensibilité réglée en dernier lieu et diminué de la marge (Y) de sécurité supplémentaire éventuellement présente, et que, si ce n'est pas le cas, lors du réglage automatique de la sensibilité (S) du premier dispositif de détection (27), on utilise comme valeur minimale (T) déterminée en dernier lieu pour la sensibilité la sensibilité diminuée de la marge (Y) de sécurité supplémentaire éventuellement présente.

16. Procédé suivant l'une des revendications 9 à 13, **caractérisé en ce que** dans le cas où à un deuxième point dans le temps le battement cardiaque naturel détecté par le deuxième dispositif de détection (43) n'est pas également détecté au moyen du premier dispositif (27) de détection, lors du réglage automatique de la sensibilité (S) du premier dispositif (27) de détection, on utilise, comme valeur minimale (T) déterminée en dernier lieu de la sensibilité (S) la valeur réglée en dernier lieu de la sensibilité.

17. Procédé suivant l'une des revendications 9 à 13 ou 16, **caractérisé en ce que** dans le cas où un battement cardiaque naturel détecté au moyen du deuxième dispositif (43) de détection n'est pas aussi détecté au moyen du premier dispositif (27) de détection, la sensibilité (S) du premier dispositif (27) de détection est réglée à sa valeur (Sₘₐₓ) maximale pour la détection du battement cardiaque naturel suivant.
